# EUROPEAN PATENT APPLICATION

(11) **EP 4 112 721 A1**
(43) Date of publication of application: **04.01.2023**
(21) Application number: 21821147.2
(22) Date of filing: 10.06.2021
(51) Int. Cl.: C12N 5/10, A61P 35/00

(54) **ENGINEERED IMMUNE CELL EXPRESSING NK INHIBITORY MOLECULE AND USE THEREOF**

(30) Priority: 11.06.2020 CN 202010527572; 03.11.2020 CN 202011209420
(71) Applicant: Nanjing Bioheng Biotech Co., Ltd, Nanjing, Jiangsu 210061 (CN)
(72) Inventor: ZHOU, Yali, Nanjing, Jiangsu 210061 (CN); CHEN, Gong, Nanjing, Jiangsu 210061 (CN); JIANG, Xiaoyan, Nanjing, Jiangsu 210061 (CN); REN, Jiangtao, Nanjing, Jiangsu 210061 (CN); HE, Xiaohong, Nanjing, Jiangsu 210061 (CN); WANG, Yanbin, Nanjing, Jiangsu 210061 (CN); HAN, Lu, Nanjing, Jiangsu 210061 (CN)
(74) Representative: Comoglio, Elena
(86) International application number: PCT/CN2021/099314
(87) International publication number: WO 2021/249462

(57) **Abstract**

Provided is an NK inhibitory molecule, which includes one or more NK inhibitory ligands, a transmembrane domain and a co-stimulatory domain, wherein the NK inhibitory ligand specifically binds to NK inhibitory receptors to inhibit the killing of NK cells against the engineered immune cells expressing the NK inhibitory molecule. Also provided is an engineered immune cell expressing the NK inhibitory molecule of the present invention, wherein the expression of at least one MHC-related gene is suppressed or silenced. Further provided is the use of the engineered immune cell in the treatment of cancers, infections or autoimmune diseases. Compared to the traditional engineered immune cells, the engineered immune cell can significantly inhibit the killing effect of NK cells in a subject, thereby reducing the risk of HvGD.

## Description

### Technical Field

The present disclosure belongs to the field of immunotherapy. More specifically, the present disclosure relates to an NK inhibitory molecule comprising one or more NK inhibitory ligands, a transmembrane domains, and a co-stimulatory domain, wherein the NK inhibitory ligands specifically bind to NK inhibitory receptors to inhibit the killing of NK cells against the engineered immune cells expressing the NK inhibitory molecule.

### Background Art

In recent years, cancer immunotherapy technology has been rapidly developed, and particularly immunotherapy associated with chimeric antigen receptor T cell (CAR-T) has achieved excellent clinical effects in the treatment of hematologic tumors. CAR-T cell immunotherapy is to genetically modify T cells *in vitro* to enable the T cells to recognize tumor antigens, and the T cells, after being amplified to a certain amount, are infused back into the patients' body to kill the cancer cells, thus achieving the purpose of treating tumors.

In 2017, two autologous CAR-T therapies were approved by the FDA and launched in US, one for B-cell acute leukemia and the other for diffuse B-cell non-Hodgkin's lymphoma. Although these two CAR-T cells have excellent clinical treatment effects, they are expensive and have a long preparation cycle, making the large-scale promotion quite difficult. Therefore, it is necessary to develop a universal CAR-T product to solve the above problems. Universal CAR-T can be prepared by using T cells isolated from peripheral blood of healthy donors, thereby realizing allogeneic transfusion, and greatly shortening the waiting time of a patient for treatment. Besides, the viability and function of T cells obtained from healthy donors are also superior to those of patient-derived T cells, which can increase the CAR infection rate and improve the therapeutic effect.

However, the development of universal CAR-T cells still faces two problems:
(1) after the engineered CAR-T cells enter a patient's body and proliferate to a certain extent, they may attack normal cells or tissues of the patient, thus generating graft-versus-host disease (GvHD); and (2) the normal immune system in the patient's body may reject exogenous CAR-T cells, thus causing host-versus-graft disease (HvGD). Currently, HvGD is reduced or avoided mainly by knocking out CD52 or HLA Specifically, knocking out CD52 can make the universal CAR-T cells resistant to Alemtuzumab (CD52 antibody), thereby avoiding killing of the introduced CAR-T cells when Alemtuzumab is used to clear the T cells of the patient. However, using Alemtuzumab will increase the production and treatment costs of the universal CAR-T product. On the other hand, although knocking out HLA molecule can prevent CAR-T cells from killing by the patient's T cells without using an antibody or other treatments, cells with HLA molecule being knocked out will be recognized by NK cells of the patient and cause rejection reaction.

Therefore, the existing universal CAR cell therapy still needs to be improved, especially reducing the killing effect of NK cells against CAR cells, thereby further reducing or avoiding the risk of HvGD.

### Summary

In the first aspect, the present disclosure provides an NK inhibitory molecule, which contains one or more NK inhibitory ligands, a transmembrane domain, and a co-stimulatory domain, wherein the NK inhibitory ligands specifically bind to NK inhibitory receptors (NKIR) to inhibit the killing of NK cells against engineered immune cells expressing the NK inhibitory molecule.

In an embodiment, the NK inhibitory ligand is an antibody targeting NKIR or a functional fragment thereof, or a natural ligand of NKIR or an NKIR binding fragment contained therein. In an embodiment, the NKIR is selected from the group consisting of an NKG2/CD94 component (e.g., NKG2A, NKG2B, CD94); a killer cell Ig-like receptor (KIR) family member (e.g., KIR2DL1, KIR2DL2/3, KIR2DL5A, KIR2DL5B, KIR3DL1, KIR3DL2, and KIR3DL3); a leukocyte Ig-like receptor (LIR) family member (e.g., LIR1, LIR2, LIR3, LIR5, and LIR8); an NK cell receptor protein 1 (NKR-P1) family member (e.g., NKR-P1 Band NKR-P1D); an immune checkpoint receptor (e.g., PD-1, TIGIT and CD96, TIM3, LAG3); a carcinoembryonic antigen-related cellular adhesion molecule 1 (CEACAM1); a sialic acid-binding immunoglobulin-like lectin (SIGLEC) family member (e.g., SIGLEC7 and SIGLEC9); a leukocyte-associated immunoglobulin-like receptor 1 (LAIR1); an Ly49 family member (e.g. Ly49A, Ly49C, Ly49F, Ly49G1, and Ly49G4), and a killer cell lectin-like receptor G1 (KLRG1). Preferably, the NKIR is selected from the group consisting of PD1, NKG2A, NKG2B, CD94, LIR1, LIR2, LIR3, KIR2DL1, KIR2DL2/3, KIR3DL1, CEACAM1, LAIR1, SIGLEC7, SIGLEC9, and KLRG1. More preferably, the NKIR is selected from the group consisting of PD1, NKG2A, CD94, KIR2DL1, KIR2DL2/3, KIR3DL1, LIR1, CEACAM1, LAIR1, SIGLEC7, SIGLEC9, and KLRG1.

In an embodiment, the NK inhibitory ligand is an antibody targeting NKIR, and the antibody is an intact antibody, Fab, Fab', F(ab')2, Fv fragment, scFv antibody fragment, linear antibody, sdAb or nanobody. In a preferred embodiment, the NK inhibitory ligand is an antibody targeting PD1, NKG2A, LIR1, KIR, SIGLEC7, SIGLEC9 and/or KLRG1 or a functional fragment thereof.

In an embodiment, the NK inhibitory ligand is a natural ligand of NKIR or an NKIR binding fragment contained therein. Preferably, the NK inhibitory ligand is selected from the group consisting of HLA-E, HLA-F, HLA-G, cadherin, collagen, OCIL, sialic acid, immune checkpoint ligand (such as PD-L1/PD-L2, CD155, CD112, CD113, Gal-9, and FGL1), and an NKIR binding region contained therein. More preferably, the NK inhibitory ligand is sialic acid, HLA-E, HLA-F, HLA-G, cadherin, PD-L1, PD-L2 or an NKIR binding region contained therein. More preferably, the NK inhibitory ligand is selected from the group consisting of sialic acid, HLA-E extracellular region, HLA-G extracellular region, E-cadherin extracellular region, PD-L1 extracellular region, and PD-L2 extracellular region. More preferably, the NK inhibitory ligand is an E-cadherin extracellular region comprising EC1 and EC2, and more preferably comprising EC1, EC2, EC3, EC4, and EC5.

In an embodiment, the transmembrane domain comprised in the NK inhibitory molecule is a transmembrane domain of a protein selected from the group consisting of: TCR α chain, TCR β chain, TCR γ chain, TCR δ chain, CD3 ζ subunit, CD3 ε subunit, CD3 γ subunit, CD3 δ subunit, CD45, CD4, CD5, CD8 α, CD9, CD16, CD22, CD33, CD28, CD37, CD64, CD80, CD86, CD134, CD137, CD154, and the transmembrane domain of an NKIR natural ligand, such as HLA-E, HLA-F, HLA-G, cadherin, collagen, and OCIL. In a preferred embodiment, the transmembrane domain is selected from a transmembrane domain of CD 8 α, CD4, CD28, and CD278.

In an embodiment, the transmembrane domain has at least 70%, preferably at least 80%, more preferably at least 90%, 95%, 97% or 99% or 100% sequence identity to an amino acid sequence represented by SEQ ID NO: 9 or 11.

In an embodiment, the NK inhibitory molecule comprises a co-stimulatory domain, which is a co-stimulatory signaling domain of a protein selected from the group consisting of LTB, CD94, TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134 (OX40), CD137 (4-1BB), CD270 (HVEM), CD272 (BTLA), CD276 (B7-H3), CD278 (ICOS), CD357 (GITR), DAP10, DAP12, LAT, NKG2C, SLP76, PD-1, LIGHT, TRIM, ZAP70, and a combination thereof. Preferably, the co-stimulatory domain of the present disclosure is from 4-1 BB, CD28, CD27, OX40, CD278 or a combination thereof.

In an embodiment, the co-stimulatory domain has at least 70%, preferably at least 80%, more preferably at least 90%, 95%, 97% or 99% or 100% sequence identity to an amino acid sequence represented by SEQ ID NO: 13 or 15.

In an embodiment, the NK inhibitory molecule does not comprise an intracellular signaling domain. In another embodiment, the NK inhibitory molecule further comprises an intracellular signaling domain.

In an embodiment, the intracellular signaling domain is a signaling domain of a protein selected from the group consisting of: FcR γ, FcR β, CD3 γ, CD3 δ, CD3 ε, CD3 ζ, CD22, CD79a, CD79b, and CD66d. Preferably, the intracellular signaling domain comprises the signaling domain of CD3 ζ.

In an embodiment, the intracellular signaling domain has at least 70%, preferably at least 80%, more preferably at least 90%, 95%, 97% or 99% or 100% sequence identity to an amino acid sequence represented by SEQ ID NO: 17 or 19.

The present disclosure further provides a nucleic acid encoding the above NK inhibitory molecule, and a vector containing the nucleic acid.

In the second aspect, the present disclosure provides an engineered immune cell, characterized in that it (1) expresses the NK inhibitory molecule of the present disclosure, and (2) the expression of at least one MHC-related gene is suppressed or silenced. In an embodiment, the engineered immune cell of the present disclosure further expresses a chimeric antigen receptor, wherein the chimeric antigen receptor contains a ligand binding domain, a transmembrane domain, a co-stimulatory domain, and an intracellular signaling domain.

In one aspect, the present disclosure provides an engineered immune cell, characterized in that it (1) expresses a fusion protein of the NK inhibitory molecule of the present disclosure and a chimeric antigen receptor, wherein the fusion protein comprises an NK inhibitory ligand, a ligand binding domain, a transmembrane domain, a co-stimulatory domain, and an intracellular signaling domain, and (2) the expression of at least one MHC-related gene issuppressed or silenced.

In an embodiment, the MHC-related gene is selected from the group consisting of HLA-A, HLA-B, HLA-C, B2M, HLA-DPA, HLA-DQ, HLA-DRA, TAP1, TAP2, LMP2, LMP7, RFX5, RFXAP, RFXANK, CIITA, and a combination thereof, preferably HLA-A, HLA-B, HLA-C, B2M, RFX5, RFXAP, RFXANK, CIITA, and a combination thereof.

In an embodiment, the expression of at least one TCR/CD3 gene of the engineered immune cell is suppressed or silenced, and the TCR/CD3 gene is selected from the group consisting of TRAC, TRBC, CD3 γ, CD3 δ, CD3 ε, CD3 ζ, and a combination thereof.

In a preferred embodiment, expression of at least one TCR/CD3 gene and at least one MHC-related gene of the engineered immune cell is suppressed or silenced, wherein the at least one TCR/CD3 gene is selected from the group consisting of TRAC, TRBC, and a combination thereof, and the at least one MHC-related gene is selected from the group consisting of B2M, RFX5, RFXAP, RFXANK, CIITA, and a combination thereof. In an embodiment, expression of TRAC or TRBC and expression of B2M of the engineered immune cell are suppressed or silenced. In an embodiment, expression of TRAC or TRBC and expression of CIITA of the engineered immune cell are suppressed or silenced. In a preferred embodiment, expression of TRAC or TRBC, and expression of B2M and CIITA of the engineered immune cell are suppressed or silenced. In a preferred embodiment, expression of TRAC or TRBC, and expression of B2M and RFX5 of the engineered immune cell are suppressed or silenced.

In an embodiment, the engineered immune cell of the present disclosure is further characterized in that expression of one or more genes is suppressed or silenced, wherein the one or more genes are selected from the group consisting of CD52, GR, dCK, and immune checkpoint gene, such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3. Preferably, CD52, dCK, PD1, LAG3, TIM3, CTLA4, TIGIT or a combination thereof of the engineered immune cell is suppressed or silenced.

In an embodiment, the ligand binding domain binds to a target selected from the group consisting of: TSHR, CD2, CD3, CD4, CD5, CD7, CD8, CD14, CD15, CD19, CD20, CD21, CD23, CD24, CD25, CD37, CD38, CD40, CD40L, CD44, CD46, CD47, CD52, CD54, CD56, CD70, CD73, CD80, CD97, CD123, CD22, CD126, CD138, CD 179a, DR4, DR5, TAC, TEM1/CD248, VEGF, GUCY2C, EGP40, EGP-2, EGP-4, CD133, IFNAR1, DLL3, kappa light chain, TIM3, tEGFR, IL-22Ra, IL-2, ErbB3, ErbB4, MUC16, MAGE-A3, MAGE-A6, NKG2DL, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38, CD44v6, CEA, EPCAM, B7H3, KIT, IL-13Ra2, mesothelin, IL-11Ra, PSCA, PRSS21, VEGFR2, LewisY, CD24, PDGFR-β, SSEA-4, CD20, AFP, Folate receptor α, ERBB2 (Her2/neu), MUC1, EGFR, CS1, CD138, NCAM, Claudin18.2, Prostase, PAP, ELF2M, Ephrin B2, IGF-I receptor, CAIX, LMP2, gp100, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE-A4, MART-1, WT-1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGFβ, APRIL, NKG2D, and any combination thereof.

In an embodiment, the engineered immune cell is a B cell, a T cell, a macrophage, a dendritic cell, a monocyte, an NK cell, or an NKT cell. Preferably, the engineered immune cell is a T cell, for example, a CD4+/CD8+ T cell, a CD4+ helperT cell (for example, Th1 and Th2 cells), a CD8+ T cell (for example, a cytotoxic T cell), a tumor infiltrating cell, a memory T cell, a naive T cell, a γδ-T cell, and an αβ-T cell.

In one aspect, the present disclosure further provides a pharmaceutical composition, which contains the NK inhibitory molecule, the nucleic acid molecule, the vector or the engineered immune cell of the present disclosure as an active agent, and one or more pharmaceutically acceptable excipients.

In one aspect, the present disclosure further provides a method of treating a subject with cancer, infection or autoimmune disease, including administering to the subject an effective amount of the NK inhibitory molecule, the nucleic acid molecule, the vector, the engineered immune cell or the pharmaceutical composition according to the present disclosure. Therefore, the present disclosure further encompasses use of the NK inhibitory molecule, the nucleic acid molecule, the vector or the engineered immune cell in the preparation of a medicine for the treatment of cancer, infection or autoimmune disease.

In an embodiment, the cancer is a solid tumor or a hematologic tumor. More specifically, the cancer is selected from the group consisting of: brain glioma, blastoma, sarcoma, leukemia, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of digestive system, endometrial cancer, esophageal cancer, eye cancer, head and neck cancer, stomach cancer, glioblastoma (GBM), liver cancer, hepatoma, intraepithelial tumor, kidney cancer, larynx cancer, liver tumor, lung cancer, lymphoma, melanoma, myeloma, neuroblastoma, oral cancer, ovarian cancer, pancreatic cancer, prostate cancer, retinoblastoma, rhabdomyosarcoma, rectal cancer, cancer of respiratory system, salivary gland cancer, skin cancer, squamous cell carcinoma, stomach cancer, testicular cancer, thyroid cancer, uterine or endometrial cancer, malignant tumor of urinary system, vulval cancer and other cancer and sarcoma, and B cell lymphoma, T cell lymphoma, mantle cell lymphoma, AIDS-related lymphoma, and Waldenstrom macroglobulinemia, chronic lymphocytic leukemia (CLL), acute lymphocytic leukemia (ALL), B cell acute lymphocytic leukemia (B-ALL), T cell acute lymphocytic leukemia (T-ALL), B cell prolymphocytic leukemia, blast cell plasmacytoid dendritic cell tumor, Burkitt lymphoma, diffuse large B cell lymphoma, follicular lymphoma, chronic myelogenous leukemia (CML), malignant lymphoproliferative disorder, MALT lymphoma, hairy cell leukemia, marginal zone lymphoma, multiple myeloma, myelodysplasia, plasmablastic lymphoma, preleukemia, plasmacytoid dendritic cell tumor, and post-transplant lymphoproliferative disorder (PTLD).

In an embodiment, the infection includes, but is not limited to, infections caused by viruses, bacteria, fungi, and parasites.

In an embodiment, the autoimmune disease includes, but is not limited to, type I diabetes, celiac disease, Graves disease, inflammatory bowel disease, multiple sclerosis, psoriasis, rheumatoid arthritis, Addison disease, sicca syndrome, Hashimoto thyroiditis, myasthenia gravis, vasculitis, pernicious anemia, and systemic lupus erythematosus, etc.

The present disclosure is advantageous in that compared with expressing NK inhibitory ligands alone, the NK inhibitory molecule of the present disclosure further comprises a co-stimulatory domain, which can further reduce/inhibit the killing of engineered immune cell by the NK cell in a subject's body. Further, when the NK inhibitory molecule comprises an intracellular signaling domain, it can enhance the killing of NK cells in the subject's body by the engineered immune cell, thereby better reducing the risk of HvGD, and realizing real allogeneic transfusion.

### Detailed Description

Unless otherwise indicated, all scientific and technical terms used herein have the same meaning as commonly understood by those of ordinary skill in the art to which the present disclosure belongs.

### NK inhibitory molecule

It has been reported that a cell in which expression of one or more HLA-class I molecules is reduced or eliminated may be identified as non-self by NK cells, thereby being killed in a targeted manner. Thus, expression of one or more NK inhibitory molecules on the cell can protect it from killing by the NK cells.

Therefore, in a first aspect, the present disclosure provides an NK inhibitory molecule, which contains one or more NK inhibitory ligands, transmembrane domains, and co-stimulatory domains, wherein the NK inhibitory ligands specifically bind to NK inhibitory receptors (NKIR) so as to inhibit the killing of NK cells against the engineered immune cell expressing the NK inhibitory molecule.

As used herein, the term "NK inhibitory ligand" refers to a molecule capable of binding to NKIR and inhibiting NK cell function (e.g., killing function). In an embodiment, the NK inhibitory ligand is an antibody targeting NKIR or a functional fragment thereof, or a natural ligand of NKIR or an NKIR binding fragment contained therein. Non-limiting examples of NKIR include NK cell surface receptors having or binding to an immunoreceptor tyrosine-based inhibitory motif (ITIM). Such receptors include, but are not limited to, an NKG2/CD94 component (e.g., NKG2A, NKG2B, CD94); a killer cell Ig-like receptor (KIR) family member (e.g., KIR2DL1, KIR2DL2/3, KIR2DL5A, KIR2DL5B, KIR3DL1, KIR3DL2, and KIR3DL3); a leukocyte Ig-like receptor (LIR) family member (e.g., LIR1, LIR2, LIR3, LIR5, and LIR8); an NK cell receptor protein 1 (NKR-P1) family member (e.g., NKR-P1 B and NKR-P1 D); an immune checkpoint receptor (e.g., PD-1, TIGIT and CD96, TIM3, LAG3); carcinoembryonic antigen-related cellular adhesion molecule 1 (CEACAM1); a sialic acid-binding immunoglobulin-like lectin (SIGLEC) family member (e.g., SIGLEC7 and SIGLEC9); a leukocyte-associated immunoglobulin-like receptor 1 (LAIR1); an Ly49 family member (e.g. Ly49A, Ly49C, Ly49F, Ly49G1, and Ly49G4), and a killer cell lectin-like receptor G1 (KLRG1).

In an embodiment, the NK inhibitory ligand is an antibody targeting NKIR or a functional fragment thereof, for example, monoclonal antibody, polyclonal antibody, recombinant antibody, human antibody, humanized antibody, murine antibody, chimeric antibody and a functional fragment thereof. Examples of antibody or functional fragments thereof include, but are not limited to, intact antibody, Fab, Fab', F(ab')2, Fv fragment, scFv antibody fragment, linear antibody, sdAb (VH or VL), nanobody (Nb), and so on, preferably selected from Fab, scFv, sdAb, and nanobody.

In an embodiment, the NK inhibitory ligand is an antibody targeting NKG2A/CD94 component or a functional fragment thereof. In a preferred embodiment, the NK inhibitory ligand is an antibody targeting NKG2A, NKG2B or CD94. NKG2/CD94 is a heterodimer composed of CD94 bound to another NKG2 subunit by a disulfide bond. The CD94 cytoplasmic domain has only seven amino acid residues, and does not have a structure for signal transmission. The NKG2 family includes members such as NKG2A, NKG2B, NKG2C, NKG2D, NKG2E, and NKG2F, wherein NKG2A and NKG2B are different spliced products of the same gene sharing a high homology. The cytoplasmic region tail of NKG2A/2B contains 2 ITIMs, and delivers inhibitory signals by recruiting SHP1 or SHP-2. The natural ligand of NKG2A/2B is HLA-E. As the binding affinity of NKG2A/2B to the ligand is higher than that of the activated receptor NKG2C, when both the inhibitory receptor and the activation receptor of NK cells can bind to target cells expressing HLA-E, inhibitory NKG2A/CD94 will dominate, and ultimately inhibit NK cell activity. NKG2A antibody known to those skilled in the art can be used in the present disclosure, e.g., Z270 (available from Immunotech, France), Z199 (available from Beckman Coulter, USA), 20D5 (available from BD Biosciences Pharmingen, USA), P25 (available from Morettaetal, Univ. Genova, Italy), etc.

In an embodiment, the NK inhibitory ligand is an antibody targeting NKG2A, which contains (1) CDR-L1 as represented by SEQ ID NO: 72, CDR-L2 as represented by SEQ ID NO: 73, CDR-L3 as represented by SEQ ID NO: 74, CDR-H1 as represented by SEQ ID NO: 75, CDR-H2 as represented by SEQ ID NO: 76, and CDR-H3 as represented by SEQ ID NO: 77, or (2) CDR-L1 as represented by SEQ ID NO: 78, CDR-L2 as represented by SEQ ID NO: 79, CDR-L3 as represented by SEQ ID NO: 80, CDR-H1 as represented by SEQ ID NO: 81, CDR-H2 as represented by SEQ ID NO: 82, and CDR-H3 as represented by SEQ ID NO: 83. In an embodiment, the NK inhibitory ligand is an antibody targeting NKG2A, which contains a light chain variable region and a heavy chain variable region, the light chain variable region has at least 70%, preferably at least 80%, more preferably at least 90%, 95%, 97% or 99% or 100% sequence identity to an amino acid sequence represented by SEQ ID NO: 3, 7 or 68, the heavy chain variable region has at least 70%, preferably at least 80%, more preferably at least 90%, 95%, 97% or 99% or 100% sequence identity to an amino acid sequence represented by SEQ ID NO: 1, 5, 67. In a preferred embodiment, the NK inhibitory ligand is an anti-NKG2A antibody containing SEQ ID NOS: 1 and 3, an anti-NKG2A antibody containing SEQ ID NOS: 5 and 7 or an anti-NKG2A antibody containing SEQ ID NOS: 67 and 68.

In an embodiment, the NK inhibitory ligand is an antibody targeting KIR or a functional fragment thereof. The KIR molecule is a type I transmembrane protein, and belongs to the immunoglobulin superfamily, and the structure thereof includes an extracellular region, a transmembrane region, and a cytoplasmic region. According to the number of Ig-like domains contained in the extracellular region, KIR can be divided into KIR2D and KIR3D subfamilies. According to the length of cytoplasmic region, KIR further may be divided into long forms (L) and short forms (S), such as KIR2DL, KIR2DS, KIR3DL, and KIR3DS. In the above, the cytoplasmic region of KIR2DL1, KIR2DL2, KIR2DL3, KIR3DL1, KIR3DL2, and KIR3DL3 contains two immunoreceptor tyrosine-based inhibitory motifs (ITIM), and the cytoplasmic region of KIR2DL5 contains one ITIM, and they are inhibitory KIR receptors. Specifically, the phosphorylation of ITIM contained in the cytoplasmic region of the inhibitory KIR receptor is phosphorylated will recruit the phosphatases SHP1 and SHP2, causing dephosphorylation of the cellular substrate, and finally inhibiting or terminating the effector functions of NK cells, such as cytotoxicity and cytokine secretion. KIR is expressed on most of NK cells, although there are differences in expression levels in different individuals. Even in the same individual, types of KIRs expressed by different NK cells are not all the same, and the same NK cell can express several different KIR molecules. Recognition ligands of KIR are classical HLA-class I molecules, including certain polymorphic epitopes of HLA-A, HLA-B, and HLA-C. For example, KIR3DL2 recognizes HLA-A alleles-A3 and -A11, KIR3DL1 recognizes HLA-Bw-4, KIR2DL1 recognizes HLA-Cw2, HLA-Cw4, and HLA-Cw6 isoforms. The mouse homologue of KIR is gp49B1, which is 335 amino acids in length, and contains 2 ITIM structures in the cytoplasmic region. In a preferred embodiment, the antibody targeting KIR is an antibody targeting one or more targets selected from the group consisting of: KIR2DL1, KIR2DL2, KIR2DL3, KIR3DL1, KIR3DL2, KIR3DL3, KIR2DL5, and gp49B1. KIR antibodies that are well known to those skilled in the art can be used in the present disclosure, e.g., GL183 (targeting KIR2DL2/L3, available from Immunotech, France and Beckton Dickinson, USA), EB6 (targeting KIR2DL1, available from Immunotech, France, and Beckton Dickinson, USA), AZ138 (targeting KIR3DL1, available from Morettaetal, Univ. Genova, Italy), Q66 (targeting KIR3DL2, available from Immunotech, France), Z27 (targeting KIR3DL1, available from Immunotech, France and Beckton Dickinson, USA), etc.

In an embodiment, the NK inhibitory ligand is an antibody targeting KIR, which comprises CDR-L1 as represented by SEQ ID NO: 84, CDR-L2 as represented by SEQ ID NO: 85, CDR-L3 as represented by SEQ ID NO: 86, CDR-H1 as represented by SEQ ID NO: 87, CDR-H2 as represented by SEQ ID NO: 88, and CDR-H3 as represented by SEQ ID NO: 89. In an embodiment, the NK inhibitory ligand is an antibody targeting KIR, which comprises a light chain variable region and a heavy chain variable region, the light chain variable region has at least 70%, preferably at least 80%, more preferably at least 90%, 95%, 97% or 99% or 100% sequence identity to an amino acid sequence represented by SEQ ID NO: 58, and the heavy chain variable region has at least 70%, preferably at least 80%, more preferably at least 90%, 95%, 97% or 99% or 100% sequence identity to an amino acid sequence represented by SEQ ID NO: 59. In a preferred embodiment, the NK inhibitory ligand is an anti-KIR antibody comprising SEQ ID NO: 58 and SEQ ID NO: 59, and an amino acid sequence thereof is, for example, as represented by SEQ ID NO: 57 or 60.

In an embodiment, the NK inhibitory ligand is an antibody targeting LIR or a functional fragment thereof. LIR is also known as immunoglobulin-like transcripts (ILT) or monocyte-macrophage inhibitory receptor (MIR). The LIR family has 8 members, in which the cytoplasmic regions of LIR1 (also referred to as I LT2), LIR2 (also referred to as ILT4), LIR3 (also referred to as ILT5), LIR5 (also referred to as ILT3), and LIR8 contain 2-4 ITIM structures, among which at least one is a VXYXXL/V motif, and they are inhibitory LIR receptors. LIR-1 has been reported to be capable of inhibiting the killing of NK cell line NKL against target cell expressing HLA-class I molecules and the activation of NKL mediated by CD16. Homologue of LIR in mice is PIR (Paired Ig-like receptor), including PIR-A and PIR-B, in which PIR-A transmits activation signals with the aid of FcRγ homodimer, while PIR-B transmits inhibition signals through four ITIM structures contained in the cytoplasmic region thereof. In a preferred embodiment, the NK inhibitory ligand is an antibody targeting LIR1, LIR2, LIR3, LIR5, LIR8 or PIR-B.

In an embodiment, the NK inhibitory ligand is an antibody targeting LIR1, which comprises (1) CDR-L1 as represented by SEQ ID NO: 90, CDR-L2 as represented by SEQ ID NO: 91, CDR-L3 as represented by SEQ ID NO: 92, CDR-H1 as represented by SEQ ID NO: 93, CDR-H2 as represented by SEQ ID NO: 94, and CDR-H3 as represented by SEQ ID NO: 95, or (2) CDR-L1 as represented by SEQ ID NO: 96, CDR-L2 as represented by SEQ ID NO: 97, CDR-L3 as represented by SEQ ID NO: 98, CDR-H1 as represented by SEQ ID NO: 99, CDR-H2 as represented by SEQ ID NO: 100, and CDR-H3 as represented by SEQ ID NO: 101. In an embodiment, the NK inhibitory ligand is an antibody targeting LIR1, which comprises a light chain variable region and a heavy chain variable region, the light chain variable region has at least 70%, preferably at least 80%, more preferably at least 90%, 95%, 97% or 99% or 100% sequence identity to an amino acid sequence represented by SEQ ID NO: 61 or 65, and the heavy chain variable region has at least 70%, preferably at least 80%, more preferably at least 90%, 95%, 97% or 99% or 100% sequence identity to an amino acid sequence represented by SEQ ID NO: 62 or 64. In a preferred embodiment, the NK inhibitory ligand is an anti- LIR1 antibody having an amino acid sequence as represented by SEQ ID NO: 63 or 66. Other antibodies targeting the LIR family members known in the art can also be used in the present disclosure. In an embodiment, the NK inhibitory ligand is an antibody targeting an immune checkpoint receptor (e.g. PD-1, TIGIT, CD96, TIM3, and LAG3) or a functional fragment thereof. In a preferred embodiment, the NK inhibitory ligand is an antibody targeting PD-1 or a functional fragment thereof. PD-1 is mainly expressed in activated NK cells. It belongs to the CD28 family member, and is a type I transmembrane glycoprotein consisting of 268 amino acids. Its structure mainly includes an extracellular immunoglobulin variable domain (IgV)-like structure, a hydrophobic transmembrane region, and an intracellular region. The tail of the intracellular region has two independent tyrosine residues, wherein the tyrosine residue at the nitrogen end participates in constituting an ITIM, and the tyrosine residue at the carbon end participates in constituting an immunoreceptor tyrosine based switch motif (ITSM). PD-1 binding to its ligands (such as PD-L1 and PD-L2) promotes tyrosine in the ITSM domain of PD-1 to be phosphorylated, and further cause dephosphorylation of downstream protein kinases Syk and PI3K, and inhibition of activation of downstream AKT, ERK, and other pathways, and finally inhibit the activity of NK.

In a preferred embodiment, the NK inhibitory ligand is an antibody targeting TIGIT or a functional fragment thereof. TIGIT belongs to the immunoglobulin superfamily member, and it consists of an extracellular immunoglobulin variable region (IgV) domain, a type 1 transmembrane domain, and an intracellular domain with ITIM and immunoglobulin tyrosine tail (ITT) motif. Upon binding to a ligand (e.g., CD155, CD112, CD113), the transmission of intracellular inhibitory signal may be induced, thereby inhibiting the activity of NK cells.

In a preferred embodiment, the NK inhibitory ligand is an antibody targeting LAG3 or a functional fragment thereof. LAG3 is a member of the protein Ig superfamily. It is a type 1 transmembrane protein expressed on activated T cell, NK cell, B cell, and plasmacytoid dendritic cell. The four IgG domains of LAG3 have high structural homology to the CD4 molecule, but homology of their amino acids is lower than 20%. Studies have shown that LAG3 has a negative regulatory effect on proliferation and long-term memory of T cells and NK cells. Once activated by its ligands (e.g., FGL1), it can promote "bad cells" such as tumor cells to escape from the killing of the immune system.

In a preferred embodiment, the NK inhibitory ligand is an antibody targeting TIM3 or a functional fragment thereof. TIM3 is a receptor protein of the TIM family, and is expressed on the surface of T cell, Treg cell, innate immune cell (dendritic cell, natural killer cell, and monocyte). The TIM family members are encoded by three genes, specifically, HAVCR1 encodes TIM1, HAVCR2 encodes TIM3, and TIMD4 encodes TIM4. TIM3 has multiple ligands, such as phosphatidylserine, galectin-9(Gal-9), HMGB1, and CEACAM-1. When expressed in NK cells, TIM3 is considered as a possible marker for dysfunctional NK cells, and TIM3 blockade has been proved to be able to reverse NK cell dysfunction.

In an embodiment, the NK inhibitory ligand is an antibody targeting NKR-P1 or a functional fragment thereof. NKR-P1 is a type II transmembrane glycoprotein, and expressed in all human, mouse, and rat NK cells. At present, six NKR-P1 members of mice, i.e. NKR-P1A, NKR-P1 B, NKR-P1 C, NKR-P1 D, NKR-P1E, and NKR-P1 F, respectively, have been found, while only NKR-P1A (also referred to as CD161) is found in human body. The extracellular region of the NKR-P1 molecule is an NK receptor domain (NKD) in a C-type lectin-like superfamily, and is structurally similar to Ly49, Cd69, and CD94/NKG2 molecules. Although NKR-P1 is primarily present in a form of homodimer, human NKR-P1A may exist in a monomer form. The cytoplasmic regions of the NKR-P1 family molecules are structurally different among different species, for example, the cytoplasmic regions of NKR-P1B and NKR-P1D contain an ITIM motif, which recruits SHP-1 to transmit inhibitory signals after tyrosine phosphorylation, but NKR-P1C binds to the Fc receptor through a positively charged amino acid in the transmembrane region, and then recruits Syk to transmit an activation signal. Therefore, in a preferred embodiment, the NK inhibitory ligand is an antibody targeting NKR-P1B or NKR-P1D.

In an embodiment, the NK inhibitory ligand is an antibody targeting CEACAM1 or a functional fragment thereof. CEACAM1, also known as CD66a, bile glycoprotein (BGP) or C-CAM1, is a member of the carcinoembryonic antigen (CEA) gene family and belongs to the immunoglobulin (Ig) superfamily. In activated NK cells, CEACAM1 expression is up-regulated, and its homophilic interaction causes inhibition of lymphocyte toxicity effect. CEACAM1 interacts with other known CEACAM proteins including CD66a (CEACAM1), CD66c (CEACAM6), and CD66e (CEACAM5, CEA) proteins. In human, 11 different CEACAM1 splice variants have been detected to date. The designation of CEACAM1 isotype is related to the number of extracellular immunoglobulin-like domains (e.g., CEACAM1 with 4 extracellular immunoglobulin-like domains is referred to as CEACAM1-4), and the length of the cytoplasmic tail (e.g., CEACAM1-4 with a long cytoplasmic tail is referred to as CEACAM1-4L, and CEACAM1-4 with a short cytoplasmic tail is referred to as CEACAM1-4S). The N-terminal domain of CEACAM1 begins immediately after a signal peptide, and its structure is considered as an IgV-type.

In an embodiment, the NK inhibitory ligand is an antibody targeting SIGLEC or a functional fragment thereof. 16 SIGLEC proteins have been identified in human and 9 SIGLEC proteins have been identified in mice, wherein the SIGLEC proteins consist of 2-17 extracellular Ig domains including amino-terminal V-type domains comprising a sialic acid binding site. Siglecs are typically divided into two groups: a first subset consisting of Siglec1, Siglec2, Siglec4, and Siglec15, and a CD33-related second subset including Siglec3, Siglec5, Siglec6, Siglec7, Siglec8, Siglec9, Siglec10, Siglec11, Siglec12, Siglec14, and Siglec16. Siglec7, also known as p75, CD328 or AIRM, comprises an extracellular N-terminal Ig-like V-type domain, two Ig-like C2-type domains, and an intracellular region containing one ITIM motif and one ITIM-like motif. Siglec7 is constitutively expressed on NK cells, dendritic cells, monocytes, and neutrophils. Siglec7 has been observed to have an inhibitory effect on NK cell-mediated tumor clearance. The structure of Siglec9 is quite similar to Siglec7, their N-terminal V-group Ig domains have overall amino acid sequence identity of about 77%, and show different sialic acid binding specificities. In view of functional studies on NK cells, tumor cells expressing Siglec9-binding sialic acid ligand have been proved to inhibit NK cell activation and tumor cell killing. Many human tumors robustly up-regulate sialic acid ligand that binds to Siglec9, which allows the tumors to be capable of escaping immunity and then cancer progression occurs.

In a preferred embodiment, the NK inhibitory ligand is an antibody targeting Siglec7 or Siglec9, such as those known in the art. By way of example, an anti-Siglec7 antibody may be derived from human Siglec7/CD328 antibody (AF1138, R&D Systems), clone #194212 (MAB1138, R&D Systems), clone #194211 (MAB11381, R&D Systems), clone Z176 (A22330, Beckman Coulter), 6-434 (339202, Biolegend), REA214 (Miltenyl Biotec), S7.7 (MCA5782GA, BioRad), 10B2201 (MBS604764, MyBioSource), 8D8 (MBS690562, MyBioSource), 10B2202 (MBS608694, MyBioSource), and 5-386 (MBS214370, MyBioSource). The anti-Siglec9 antibody can be derived from MAB1139 (clone #191240, R&D Systems), AF1139 (R&D Systems), D18 (SC-34936, Santa Cruz Biotechnology), Y-12SC34938 (SC3-4938, Santa Cruz Biotechnology), AB 197981 (Abeam), AB96545 (Abeam), AB89484 (clone #MM0552-6K12, Abeam), AB 130493 (Abeam), AB117859 (clone #3G8, Abeam), E10-286 (Becton Dickinson). Given the similarity in extracellular structure of Siglec7 and Siglec9, antibodies that simultaneously target the two may also act as a NK inhibitory ligand of the present disclosure.

In an embodiment, the NK inhibitory ligand is an antibody targeting SIGLEC7, SIGLEC9 or both, which comprises (1) CDR-L1 as represented by SEQ ID NO: 102, CDR-L2 as represented by SEQ ID NO: 103, CDR-L3 as represented by SEQ ID NO: 104, CDR-H1 as represented by SEQ ID NO: 105, CDR-H2 as represented by SEQ ID NO: 106, and CDR-H3 as represented by SEQ ID NO: 107, (2) CDR-L1 as represented by SEQ ID NO: 122, CDR-L2 as represented by SEQ ID NO: 123, CDR-L3 as represented by SEQ ID NO: 124, CDR-H1 as represented by SEQ ID NO: 125, CDR-H2 as represented by SEQ ID NO: 126 and CDR-H3 as represented by SEQ ID NO: 127, (3) CDR-L1 as represented by SEQ ID NO: 131, CDR-L2 as represented by SEQ ID NO: 132, CDR-L3 as represented by SEQ ID NO: 133, CDR-H1 as represented by SEQ ID NO: 134, CDR-H2 as represented by SEQ ID NO: 135 and CDR-H3 as represented by SEQ ID NO: 136, (4) CDR-L1 as represented by SEQ ID NO: 140, CDR-L2 as represented by SEQ ID NO: 141, CDR-L3 as represented by SEQ ID NO: 142, CDR-H1 as represented by SEQ ID NO: 143, CDR-H2 as represented by SEQ ID NO: 144, and CDR-H3 as represented by SEQ ID NO: 155, (5) CDR-L1 as represented by SEQ ID NO: 176, CDR-L2 as represented by SEQ ID NO: 177, CDR-L3 as represented by SEQ ID NO: 178, CDR-H1 as represented by SEQ ID NO: 179, CDR-H2 as represented by SEQ ID NO: 180 and CDR-H3 as represented by SEQ ID NO: 181, or (6) CDR-L1 as represented by SEQ ID NO: 188, CDR-L2 as represented by SEQ ID NO: 189, CDR-L3 as represented by SEQ ID NO: 190, CDR-H1 as represented by SEQ ID NO: 191, CDR-H2 as represented by SEQ ID NO: 192, and CDR-H3 as represented by SEQ ID NO: 193. The above antibodies (1)-(4) target SIGLEC7, and antibodies (5)-(6) simultaneously target both SIGLEC7 and SIGLEC9. In an embodiment, the NK inhibitory ligand is an antibody targeting SIGLEC7, SIGLEC9 or both, which comprises a light chain variable region and a heavy chain variable region, the light chain variable region has at least 70%, preferably at least 80%, more preferably at least 90%, 95%, 97% or 99% or 100% sequence identity to an amino acid sequence represented by SEQ ID NO: 108, 128, 137, 146, 182, 185 or 194, and the heavy chain variable region has at least 70%, preferably at least 80%, more preferably at least 90%, 95%, 97% or 99% or 100% sequence identity to an amino acid sequence represented by SEQ ID NO: 109, 129, 138, 147, 183, 186 or 195. In a preferred embodiment, the NK inhibitory ligand is an antibody against SIGLEC7, SIGLEC9 or both, whose amino acid sequence is as represented by SEQ ID NO: 110, 130, 139, 148, 184, 187 or 196. In an embodiment, the NK inhibitory ligand is an antibody targeting LAIR1 or a functional fragment thereof. LAIR1 contains 10 exons, encoding a type I transmembrane glycoprotein consisting of 287 amino acids, which contains a single extracellular type C2 Ig-like domain, followed by a stem region linked to a single transmembrane domain and two ITIM motifs transmitting inhibitory signals. LAIR1 has certain structural homology to LIR and KIR family members, indicating that these molecules may be derived from the same progenitor gene. LAIR1 is expressed in T cell, B cell, natural killer (NK) cell, macrophage, and dendritic cell, and hematopoietic progenitor cell including human CD34+ cells. Due to the presence of the ITIM motif, studies in human and mice have so far found that LAIR1 plays a role in immunosuppression. Further studies show that LAIR1 not only can inhibit static NK cells, but also can inhibit the killing of activated NK cells against target cells.

In an embodiment, the NK inhibitory ligand is an antibody targeting Ly49 or a functional fragment thereof. Ly49 is a type II transmembrane glycoprotein, can form a homodimer by linkage of disulfide bond, and exerts a function similar to that of human KIR, i.e. transmitting a signal by interacting with MHC-class I molecular ligand, further adjusting the activity of NK cells. The Ly49 family of mice has been found to include 11 members, i.e. Ly49A, Ly49B, Ly49C, Ly49D, Ly49E, Ly49F, Ly49G, Ly49H, Ly49I, Ly49P, and Ly49Q. In the above, Ly49A, Ly49C, Ly49F, Ly49G, and Ly49Q all contain an ITIM motif in the cytoplasmic region, can bind to and activate the tyrosine kinase SHP-1, and inhibit the activation of NK cells by interfering with the production of phosphorylated tyrosine. Therefore, in a preferred embodiment, the NK inhibitory ligand is an antibody targeting Ly49A, Ly49C, Ly49F, Ly49G or Ly49Q.

In an embodiment, the NK inhibitory ligand is an antibody targeting KLRG1 or a functional fragment thereof. KLRG1 is a type II transmembrane protein surface co-inhibitory receptor that modulates the activity of T cells and NK cells. The extracellular portion thereof cmprises a C-type lectin domain, and the known ligand is cadherin, and an intracellular portion thereof comprises an immunoreceptor tyrosine-based inhibitory motif (ITIM) domain. It has been reported that KLRG1 receptor expression on peripheral blood NK cells of hepatitis C patients will promote the reduction of the number of NK cells and function damage, and a mechanism thereof is mainly to inhibit the proliferation of NK cells, promote the apoptosis of NK cells, and reduce the release of inflammatory cytokines of NK cells.

In an embodiment, the NK inhibitory ligand is an antibody targeting KLRG1, which comprises: (1) CDR-L1 as represented by SEQ ID NO: 111, CDR-L2 as represented by SEQ ID NO: 112, CDR-L3 as represented by SEQ ID NO: 113, CDR-H1 as represented by SEQ ID NO: 114, CDR-H2 as represented by SEQ ID NO: 115, and CDR-H3 as represented by SEQ ID NO: 116, (2) CDR-L1 as represented by SEQ ID NO: 149, CDR-L2 as represented by SEQ ID NO: 150, CDR-L3 as represented by SEQ ID NO: 151, CDR-H1 as represented by SEQ ID NO: 152, CDR-H2 as represented by SEQ ID NO: 153 and CDR-H3 as represented by SEQ ID NO: 154, (3) CDR-L1 as represented by SEQ ID NO: 158, CDR-L2 as represented by SEQ ID NO: 159, CDR-L3 as represented by SEQ ID NO: 160, CDR-H1 as represented by SEQ ID NO: 161, CDR-H2 as represented by SEQ ID NO: 162, and CDR-H3 as represented by SEQ ID NO: 163, or (4) CDR-L1 as represented by SEQ ID NO: 167, CDR-L2 as represented by SEQ ID NO: 168, CDR-L3 as represented by SEQ ID NO: 169, CDR-H1 as represented by SEQ ID NO: 170, CDR-H2 as represented by SEQ ID NO: 171, and CDR-H3 as represented by SEQ ID NO: 172. In an embodiment, the NK inhibitory ligand is an antibody targeting KLRG1, which comprises a light chain variable region and a heavy chain variable region, the light chain variable region has at least 70%, preferably at least 80%, more preferably at least 90%, 95%, 97% or 99% or 100% sequence identity to an amino acid sequence represented by SEQ ID NO: 117, 155, 164 or 173, and the heavy chain variable region has at least 70%, preferably at least 80%, more preferably at least 90%, 95%, 97% or 99% or 100% sequence identity to an amino acid sequence represented by SEQ ID NO: 118, 156, 165 or 174. In a preferred embodiment, the NK inhibitory ligand is an anti-KLRG1 antibody having an amino acid sequence represented by SEQ ID NO: 119, 157, 166 or 175.

In an embodiment, the NK inhibitory ligand is an NKIR natural ligand or an NKIR binding region (e.g., an extracellular region) contained therein, and such natural ligands include, but are not limited to, non-classical HLA-class I molecule (e.g., HLA-E, HLA-F, and HLA-G), cadherin, collagen, OCIL, sialic acid, immune checkpoint ligands (e.g., PD-L1/PD-L2, CD155, CD112, CD113, Gal-9, FGL1), and so on.

In an embodiment, the NK inhibitory ligand is a non-classical HLA-class I molecule or an extracellular region thereof, more preferably a1 and a2 domains of non-classical HLA-class I molecule. The non-classical HLA-class I molecule is located in the same chromosomal region 6p21.3, short arm of chromosome 6, consisting of a heavy chain (α chain) and a light chain (β chain, encoded by B2M gene) linked by a non-covalent bond. The α chain includes three parts, i.e. an extracellular region (including three domains a1, a2, and a3), a transmembrane domain, and a cytoplasmic region, wherein a1 and a2 form an antigen binding groove that is responsible for binding to an antigen peptide entering the groove. a3 is homologous to the constant domain of an immunoglobulin, and binds to T cell surface molecule CD8. Non-classical HLA-class I molecules include three members: HLA-E, HLA-F, and HLA-G. HLA-E regulates NK cell activity by binding to CD94/NKG2 receptors on the surface of NK cells. The function of HLA-E is to bind peptides derived from a leader sequence of HLA class I molecules (HLA-A, -B, -C, and -G), and present them to NK cells through interaction with inhibitory receptor CD94/NKG2A, thus inhibiting the lysis of cells expressing normal levels of HLA class I molecules by NK cells. Since the affinity of HLA-E to the inhibitory receptor CD94/NKG2A under physiological conditions is significantly higher than its affinity to an activating receptor CD94/NKG2C, up-regulation of the expression level of HLA-E may protect target cells from the killing effect of NK cells. HLA-F can bind to NK inhibitory receptors ILT2 and ILT4, and such binding can be effectively inhibited by ILT2 and ILT4 antibodies. HLA-F function is still being explored at present, but it is speculated that its binding to ILT2 and ILT4 may have immunosuppressive effect. HLA-G can recognize various NK inhibitory receptors, such as CD94/NKG2A, LIR-1, LIR-2, KIR2DL1, etc. It is found that HLA-G molecules on the surface of fetal cell may inhibit NK cell killing activity by binding to KIR on the surface of a parent NK cell, thereby causing the parent to be immune-tolerant to an HLA semi-heterologous fetus. In addition, HLA-G molecules highly expressed on the cell surface of solid tumors, such as melanoma, sarcoma, and lymphoma, also enable tumor cells to escape the killing, and lysis effects of NK cells.

In an embodiment, the NK inhibitory ligand is an HLA-E extracellular region, which has at least 70%, preferably at least 80%, and more preferably at least 90%, 95%, 97% or 99% or 100% sequence identity to an amino acid sequence represented by SEQ ID NO: 31 or an encoding sequence thereof has at least 70%, preferably at least 80%, and more preferably at least 90%, 95%, 97% or 99% or 100% sequence identity to a nucleotide sequence represented by SEQ ID NO: 32. In another embodiment, the NK inhibitory ligand is a mutant of the HLA-E extracellular region (containing Y84C mutation), which has at least 70%, preferably at least 80%, more preferably at least 90%, 95%, 97% or 99% or 100% sequence identity to an amino acid sequence represented by SEQ ID NO: 33, or an encoding sequence thereof has at least 70%, preferably at least 80%, more preferably at least 90%, 95%, 97% or 99% or 100% sequence identity to a nucleotide sequence represented by SEQ ID NO: 34.

In an embodiment, the NK inhibitory ligand is an HLA-G extracellular region, which has at least 70%, preferably at least 80%, and more preferably at least 90%, 95%, 97% or 99% or 100% sequence identity to an amino acid sequence represented by SEQ ID NO: 35, or an encoding sequence thereof has at least 70%, preferably at least 80%, and more preferably at least 90%, 95%, 97% or 99% or 100% sequence identity to a nucleotide sequence represented by SEQ ID NO: 36.

In an embodiment, when it is desired to knock out the endogenous B2M of the engineered immune cell and express non-classical HLA-class I molecule as NK inhibitory ligand, synonymously mutated (i.e., only the nucleotide sequence is changed while the amino acid sequence is not changed) B2M gene needs to be introduced to allow it to form complexes with non-classical HLA-class I molecules to exert inhibiting functions, meanwhile synonymously mutated B2M gene also can be protected against knockout by a gene editing tool targeting endogenous B2M. In this embodiment, the NK inhibitory ligand comprises a fusion molecule of B2M and the extracellular region of a non-classical HLA-class I molecule. For example, the NK inhibitory ligand comprises a fusion molecule of B2M and HLA-E extracellular region or HLA-G extracellular region. In a specific embodiment, the NK inhibitory ligand comprises a fusion molecule of B2M and HLA-E extracellular region, and preferably, the HLA-E extracellular region contains Y84C mutation (SEQ ID NO: 33). In a preferred embodiment, the NK inhibitory ligand comprises a presenting peptide and a fusion molecule of B2M and HLA-E extracellular region, wherein the presenting peptide is selected from SEQ ID NOS: 46-53. Methods for synonymously mutating B2M gene are well known to those skilled in the art. In a preferred embodiment, B2M has at least 70%, preferably at least 80%, more preferably at least 90%, 95%, 97% or 99% or 100% sequence identity to an amino acid sequence represented by SEQ ID NO: 37; and the nucleotide sequence of the synonymously mutated B2M gene is, for example, represented by SEQ ID NO: 38.

In an embodiment, the NK inhibitory ligand is Osteoclast inhibitory lectin (OCIL) or an NKIR binding region thereof. Mouse OCIL includes three members: OCIL (also known as Clr-b), OCILrP1 (also known as Clr-d), and mOCILrP2 (also known as Clr-g). OCIL is widely expressed in various tissues, and the expression pattern thereof is similar to that of MHC-class I molecules. OCIL is a ligand of NKR-P1 B/D. Studies have shown that expressing OCIL on tumor cells can inhibit the killing effect of NK cells against tumor cells, and OCIL-specific antibodies can reverse such inhibitory effect.

In an embodiment, the NK inhibitory ligand is cadherin or an extracellular region thereof, for example, E-cadherin (E-cad), N-cadherin (N-cad) or R-cadherin (R-cad), preferably an extracellular region of E-cadherin. Cadherins are a class of calcium-dependent transmembrane proteins that mainly mediate homogeneous intercellular adhesion, and bind to the NK inhibitory receptor KLRG1. The cadherin molecule is a type I membrane protein, consists of about 723-748 amino acids, and structurally includes an extracellular region responsible for binding ligand, a transmembrane region, and a highly conserved cytoplasmic region. The extracellular region has several cadherin repeat domains (EC), and contains repetitive sequences consisting of 4-5 amino acid residues, responsible for binding to ligands. Human E-cadherin is encoded by CDH 1 gene, and is currently the most studied member of the cadherin family. Therefore, in a preferred embodiment, the NK inhibitory ligand is an extracellular region of E-cadherin comprising EC1 and EC2. More preferably, the NK inhibitory ligand is an extracellular region of E-cadherin comprising EC1, EC2, EC3, EC4, and EC5. More preferably, the NK inhibitory ligand is E-Cad, which has at least 70%, preferably at least 80%, and more preferably at least 90%, 95%, 97% or 99% or 100% sequence identity to an amino acid sequence represented by SEQ ID NO: 39 or 41 or an encoding sequence thereof has at least 70%, preferably at least 80%, and more preferably at least 90%, 95%, 97% or 99% or 100% sequence identity to a nucleotide sequence represented by SEQ ID NO: 40 or 42.

In an embodiment, the NK inhibitory ligand is a collagen or an NKIR binding region thereof, and it binds to LAIR 1. Collagen molecule is a trimer consisting of 3 α chains, each α chain containing a (glycine-proline-hydroxyproline)n repetitive sequences. LAIR1 recognizes and interacts with the Gly-Pro-Hyp repetitive sequence. Due to the extensive presence of this repetitive sequence, it has been shown that LAIR1 can extensively bind to a variety of collagen molecules, including but not limited to transmembrane collagens, such as collagens XVII, XIII, and XXIII; and non-transmembrane collagens, such as collagen I, II, and III. Tumor cells or tumor interstitial cells often highly express a variety of collagen molecules, which may transmit inhibitory signals into immune cells by binding to the inhibitory receptor LAIR1 on the surface of immune cells, thereby achieving the purpose of immune escape.

In an embodiment, the NK inhibitory ligand is sialic acid or an NKIR binding region thereof, and it binds to an SIGLEC family member (e.g., SIGLEC7 and/or SIGLEC9). Sialic acid is an important component of the innate immune system of vertebrates, and NK cell killing activity is associated with sialylation of the surface of tumor cells. Sialylation of tumor cells not only can hinder the physical action between tumor cells and NK cells, but also can shield the activating ligands, at the surface of the tumor cells, capable of binding thereto. In addition, sialylation of the surface of the tumor cell may block the formation of immune synapses between tumor cells and NK cells, thus reducing the killing toxicity of the NK cells against the tumor. Studies have found that sialylation of the surface of tumor cells also can inhibit the killing activity of NK cells by triggering an immunosuppressive signal mediated by Siglec. Siglec7 is expressed on the surface of most of NK cells, and when sialic acid linked with α-2,8 glycosidic bonds on the surface of tumor cells binds to Siglec7 on the surface of NK cells, the activation of NK cells is inhibited, so that the tumor cells escape the killing function mediated by NK cells.

In an embodiment, the NK inhibitory ligand is PD-L1/PD-L2 or an extracellular region thereof, and it binds PD1. PD-L1 is constitutively low-expressed on antigen presenting cell (APC), and non-hematopoietic cell such as vascular endothelial cell, islet cell, and immune privilege site (such as placenta, testis, and eye). Inflammatory cytokines such as type I, and type II interferons, TNF-α, and VEGF can induce expression of PD-L1. PD-L2 is expressed only in activated macrophages and dendritic cells. Tumor cells themselves can up-regulate the expression of PD-L1, while inflammatory factors in the tumor microenvironment can also induce the expression of PD-L1 and PDL2. Up-regulated expression of PD-L1 and PD-L2 on the surface of tumor cells can trigger the transmission of immunosuppressive signals mediated by PD-1 so as to inhibit the killing activity of NK cells. In a preferred embodiment, the NK inhibitory ligand is a PD-L1 extracellular region or a PD-L2 extracellular region, which has at least 70%, preferably at least 80%, more preferably at least 90%, 95%, 97% or 99% or 100% sequence identity to an amino acid sequence represented by SEQ ID NO: 70 or 71.

In an embodiment, the NK inhibitory ligand is CD155, CD112 or CD113 or an NKIR binding region thereof, all of which bind to TIGIT. CD155 is a high affinity ligand of TIGIT. Once CD155 highly expressed on the tumor surface binds to TIGIT on the NK surface, the killing effect of NK cells against tumor cells is inhibited. CD112 and CD113 also bind to TIGIT, although the affinity is relatively weak.

In an embodiment, the NK inhibitory ligand is galectin 9 (also known as Gal-9) or an NKIR binding region thereof, and it binds to TIM3. Gal-9 is a C type lectin that is widely expressed and secreted by many hematopoietic cells, and can bind to carbohydrate moieties on cell surface proteins. On TIM3, Gal-9 binds to a carbohydrate motif on its IgV domain, and induce calcium influx and cell death of TIM3 positive NK cells. There have been a large number of proofs showing that TIM3/Gal-9 interaction plays a role in inhibiting the immune response.

In an embodiment, the NK inhibitory ligand is FGL1 or an NKIR binding region thereof, and it binds to LAG3. FGL1 belongs to the fibrinogen family, and is a newly found ligand of LAG3, but it does not have domains such as characteristic platelet binding site and thrombin sensitive site. FGL1 protein is mainly distributed in tumor cells, and tumor stromal expression is relatively low. FGL1/LAG3 interaction is another tumor immunity escape pathway independent of the B7-H1/PD-1 pathway, and blocking this pathway can achieve a synergistic effect with PD-1 pathway blockade.

As used herein, the term "transmembrane domain" refers to a polypeptide structure that enables expression of a chimeric antigen receptor on the surface of an immune cell (e.g., a lymphocyte or an NKT cell), and guides the cellular response of the immune cell against the target cell. The transmembrane domain may be natural or synthetic, and also may be derived from any membrane-bound protein or transmembrane protein. When the chimeric antigen receptor binds to the target antigen, the transmembrane domain is capable of signaling. The transmembrane domains particularly suitable for use in the present disclosure may be derived from, for example, TCR α chain, TCR β chain, TCR γ chain, TCR δ chain, CD3 ζ subunit, CD3 ε subunit, CD3 γ subunit, CD3 δ subunit, CD45, CD4, CD5, CD8 α, CD9, CD16, CD22, CD33, CD28, CD37, CD64, CD80, CD86, CD134, CD137, CD154, and a transmembrane domain of an NKIR natural ligand, such as non-classical HLA-class I molecule (such as HLA-E, HLA-F, and HLA-G), cadherin, collagen, and OCIL. Preferably, the transmembrane domain is selected from a transmembrane domain of CD8 α, CD4, CD28, and CD278. Alternatively, the transmembrane domain may be synthesized and may mainly contain a hydrophobic residue such as leucine and valine. Preferably, the transmembrane domain is derived from CD8 α or CD28, and more preferably has at least 70%, preferably at least 80%, more preferably at least 90%, 95%, 97% or 99% or 100% sequence identity to an amino acid sequence represented by SEQ ID NO: 9 or 11, or an encoding sequence of the transmembrane domain has at least 70%, preferably at least 80%, more preferably at least 90%, 95%, 97% or 99% or 100% sequence identity to a nucleotide sequence represented by SEQ ID NO: 10 or 12.

In an embodiment, the NK inhibitory molecule of the present disclosure further may contains a hinge region located between the NK inhibitory ligand and the transmembrane domain. As used herein, the term "hinge region" generally refers to any oligopeptide or polypeptide that functions to link a transmembrane domain to a ligand binding domain. Specifically, the hinge region serves to provide greater flexibility and accessibility to the ligand binding domain. The hinge region may contain up to 300 amino acids, preferably 10 to 100 amino acids and most preferably 25 to 50 amino acids. The hinge region may be completely or partially derived from a natural molecule, for example, completely or partially from the extracellular region of CD8, CD4 or CD28, or completely or partially from an antibody constant region. Alternatively, the hinge region may be a synthetic sequence corresponding to a naturally occurring hinge sequence or may be a completely synthetic hinge sequence. In a preferred embodiment, the hinge region contains a hinge region portion of CD8 α, CD28, Fc γ RIII α receptor, IgG4 or IgG1, more preferably CD8 α, CD28 or IgG4 hinge, which has at least 70%, preferably at least 80%, more preferably at least 90%, 95%, 97% or 99% or 100% sequence identity to an amino acid sequence represented by SEQ ID NO: 25, 27 or 29, or an encoding sequence of the CD28 hinge has at least 70%, preferably at least 80%, more preferably at least 90%, 95%, 97% or 99% or 100% sequence identity to a nucleotide sequence represented by SEQ ID NO: 26, 28 or 30.

The NK inhibitory molecule of the present disclosure includes a co-stimulatory domain. Therefore, when the NK inhibitory ligand binds to an NKIR, on one hand, an inhibitory signal can be transmitted to the NK cell through the signaling region of the NKIR, so as to reduce the killing thereof against the target cell (for example, the engineered immune cell of the present disclosure); on the other hand, a stimulating signal can be transmitted into the target cell (for example, the engineered immune cell of the present disclosure) through the co-stimulatory domain contained in the NK inhibitory molecule, to stimulate the proliferation and survival thereof, thus better resisting killing of the NK cell. Previous studies have found that using antibodies targeting NK cell inhibitory receptors such as NKG2A, KIR, and ILT2 can compete for binding to binding sites of inhibitory molecules such as HLA-E and HLA-G or neutralizing the inhibitory effect thereof, so as to activate the NK cells. In contrast, the present disclosure has for the first time found that expression of the NK inhibitory molecule targeting the NK inhibitory receptor can inhibit the NK cells. It is also found in the present disclosure that compared with the NK inhibitory molecule containing no co-stimulatory domain, the NK inhibitory molecule containing a co-stimulatory domain has a better inhibitory effect on the killing effect of NK cells.

The co-stimulatory domain may be an intracellular functional signaling domain from a co-stimulatory molecule, and it contains an entire intracellular portion of the co-stimulatory molecule or a functional fragment thereof. "Co-stimulatory molecule" refers to a homologous binding partner that specifically binds to a co-stimulatory ligand on a T cell, thereby mediating a co-stimulatory response (e.g. proliferation) of the T cell. The co-stimulatory molecule includes, but is not limited to, MHC class 1 molecule, BTLA, and Toll ligand receptors. Non-limiting examples of co-stimulatory domains of the present disclosure include but are not limited to a co-stimulatory signaling domain derived form a protein selected from the group consisting of LTB, CD94, TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134 (OX40), CD137 (4-1 BB), CD270 (HVEM), CD272 (BTLA), CD276 (B7-H3), CD278 (ICOS), CD357 (GITR), DAP10, DAP12, LAT, NKG2C, SLP76, PD-1, LIGHT, TRIM, ZAP70, and a combination thereof. Preferably, the co-stimulatory domain of the present disclosure is from 4-1BB, CD28, CD27, OX40, CD278 or a combination thereof, and more preferably, from 4-1BB, CD28 or a combination thereof. In an embodiment, the co-stimulatory domain of the present disclosure has at least 70%, preferably at least 80%, more preferably at least 90%, 95%, 97% or 99% or 100% sequence identity to an amino acid sequence represented by SEQ ID NO: 13 or 15, or an encoding sequence of the co-stimulatory domain has at least 70%, preferably at least 80%, more preferably at least 90%, 95%, 97% or 99% or 100% sequence identity to a nucleotide sequence represented by SEQ ID NO: 14 or 16.

In an embodiment, the NK inhibitory molecule does not comprise an intracellular signaling domain. In another embodiment, the NK inhibitory molecule further comprises an intracellular signaling domain. That is, the NK inhibitory molecule comprises an NK inhibitory ligand, a transmembrane domain, a co-stimulatory domain, and an intracellular signaling domain. In this embodiment, the binding of the NK inhibitory ligand to the NKIR can transmit an activation signal to the target cell (for example, the engineered immune cell of the present disclosure) through the co-stimulatory domain and the intracellular signaling domain, promote killing of the NK cells by the target cells, and thus further enhance the inhibitory effect on the killing effect of the NK cells.

As used herein, the term "intracellular signaling domain" refers to a protein fraction that transduces an effector function signal and guides a cell to perform a specified function. The intracellular signaling domain is responsible for intracellular primary signaling after the ligand binding domain binds to the antigen, thus causing activation of immune cell and immune reaction. In other words, the intracellular signaling domain is responsible for activating at least one of the normal effector functions of the immune cells in which the NK inhibitory molecule is expressed. For example, the effector functions of T cell can be cytolytic activity or auxiliary activity, including secretion of cytokines.

In an embodiment, the intracellular signaling domain of the present disclosure may be cytoplasmic sequences of a T cell receptor and a co-receptor, which, after antigen receptor binding, act together to initiate primary signaling, as well as any derivative or variant of these sequences and any synthetic sequence having the same or similar function. The intracellular signaling domain may comprise many immunoreceptor tyrosine-based activation motifs (ITAM). Non-limiting examples of intracellular signaling domain of the present disclosure include, but are not limited to, those derived from FcR γ, FcR β, CD3 γ, CD3 δ, CD3 ε, CD3 ζ, CD22, CD79a, CD79b, and CD66d. In a preferred embodiment, the signaling domain of the CAR of the present disclosure may contain a CD3 ζ signaling domain, and the signaling domain has at least 70%, preferably at least 80%, more preferably at least 90%, 95%, 97% or 99% or 100% sequence identity to an amino acid sequence represented by SEQ ID NO: 17 or 19, or an encoding sequence thereof has at least 70%, preferably at least 80%, more preferably at least 90%, 95%, 97% or 99% or 100% sequence identity to a nucleotide sequence represented by SEQ ID NO: 18 or 20.

In an embodiment, the NK inhibitory molecule of the present disclosure further may contain a signal peptide such that when it is expressed in a cell such as a T cell, the nascent protein is directed to the endoplasmic reticulum and subsequently to the cell surface. The core of the signal peptide may comprise a long hydrophobic amino acid segment, which has a tendency to form a single α-helix. At the end of the signal peptide, there is usually an amino acid segment recognized and cleaved by signal peptidase. The signal peptidase can cleave during or after translocation, so as to generate free signal peptide and mature protein. Then, the free signal peptide is digested by a specific protease. Signal peptides that can be used in the present disclosure are well known to those skilled in the art, for example, signal peptides derived from B2M, CD8 α, IgG1, GM-CSFR α, and so on. In an embodiment, the signal peptide that can be used in the present disclosure has at least 70%, preferably at least 80%, more preferably at least 90%, 95%, 97% or 99% or 100% sequence identity to an amino acid sequence represented by SEQ ID NO: 21 or 23, or an encoding sequence of the signal peptide has at least 70%, preferably at least 80%, more preferably at least 90%, 95%, 97% or 99% or 100% sequence identity to a nucleotide sequence represented by SEQ ID NO: 22 or 24.

### Suppression or silencing of at least one MHC-related gene

In an embodiment, the engineered immune cell expressing the NK inhibitory molecule further includes suppression or silencing of expression of at least one MHC-related gene, for example, the expression of at least one MHC gene is suppressed or silenced, or expression of a gene interacting with or modulating the expression of at least one MHC gene is suppressed or silenced.

The major histocompatibility complex (MHC) is originally characterized as a protein that plays a major role in the transplantation reaction, and it is expressed on the surface of all higher vertebrates. It is called H-2 in mice, and HLA in human cells. MHC mainly has two types: class I and class II. MHC class I protein is a heterodimer of two proteins: one is a transmembrane protein α chain encoded by MHCl gene, and the other is a β2 microglobulin chain of an extracellular protein encoded by a gene not located in the MHC gene cluster. The α chain includes three domains, and a foreign peptide binds to two most variable domains α1, α2 at N-terminal. MHC class II protein is also a heterodimer, and comprises two transmembrane proteins encoded by genes within the MHC complex. MHC class I/antigen complex interacts with cytotoxic T cells, whereas MHC class II presents antigen to helper T cells. In addition, MHC class I protein tends to be expressed in almost all nucleated cells and platelets (as well as red blood cells in mice), while MHC class II protein is more selectively expressed. Generally, MHC class II protein is expressed on B cells, some macrophages and monocytes, Langerhans cells, and dendritic cells.

The human HLA class I gene cluster contains three major loci B, C, and A. HLA-A, HLA-B, and HLA-C are HLA class I heavy chain paralogs. The class I molecule is a heterodimer consisting of MHC α heavy chain (encoded by HLA-A, HLA-B or HLA-C) and light chain (β-2 microglobulin, encoded by B2M). The heavy chain is anchored in the membrane. It is about 45 kDa, and contains 8 exons. Exon 1 encodes a leader peptide, exons 2 and 3 encode a1 and a2 domains, both of which bind to peptides, exon 4 encodes a3 domain, exon 5 encodes a transmembrane region, and exon 6 and 7 encode cytoplasmic tail. Polymorphism within exon 2 and exon 3 results in peptide binding specificity for each class of molecules. Thus, in an embodiment, suppressing or silencing MHC-related gene expression refers to suppressing or silencing the expression of one or more genes selected from the group consisting of HLA-A, HLA-B, HLA-C, and B2M.

Human HLA class II cluster also contains three major loci DP, DQ, and DR, and both class I gene cluster and class II gene cluster are polymorphic. HLA-DPA1, HLA-DQA1, and HLA-DRA belong to HLA class II α chain paralogs. The class II molecule plays a major role in the immune system by presenting an exogenous peptide, and is primarily expressed in antigen presenting cells (e.g., B lymphocyte, dendritic cell, and macrophage). Class II molecule is a heterodimer consisting of α chain and β chain both anchored in the membrane, wherein the α chain is about 33-35 kDa, and contains 5 exons. Exon 1 encodes a leader peptide, exons 2 and 3 encode two extracellular domains, exon 4 encodes a transmembrane domain, and exon 5 encodes a cytoplasmic tail. Thus, in an embodiment, suppressing or silencing MHC-related gene expression refers to suppressing or silencing the expression of one or more genes selected from the group consisting of HLA-DPA, HLA-DQ, and HLA-DRA.

Expression of MHC class I and class II also depends on a variety of accessory proteins. For example, Tap1 and Tap2 subunits are part of TAP transporter complex necessary for loading peptide antigens on the HLA class I complex. LMP2 and LMP7 proteosome subunits function in proteolytic degradation of antigens to peptides so as to be displayed on HLA. Reducing LMP7 has been shown to reduce the expression amount of MHC class I at the cell surface. MHC class II expression is induced and expressed by some positive regulators, for example, RFX complex and CIITA. The RFX complex consists of three subunits: RFXANK (also known as RFXB), RFX5, and RFX accessory protein (also known as RFXAP). The RFX complex promotes the expression of MHC class II molecules by promoting the binding of other transcription factors to the promoter of MHC class II molecules and enhancing the specificity of binding of the promoter. CIITA is a major control factor for MHC class II expression. CIITA comprises an N-terminal rich in acidic amino acids, a PST region rich in Pro, Ser, and Thr, an intermediate GTP binding region, and a C terminal rich in Leu repeat sequence (LRR), wherein the N-terminal acidic region and the PST region are transcriptional activation regions. Thus, in an embodiment, suppressing or silencing MHC-related gene expression refers to suppressing or silencing the expression of one or more genes selected from the group consisting of TAP1, TAP2, LMP2, LMP7, RFX5, RFXAP, RFXANK, and CIITA.

Thus, in an embodiment, suppressing or silencing MHC-related gene expression refers to suppressing or silencing the expression of one or more genes selected from the group consisting of HLA-A, HLA-B, HLA-C, B2M, HLA-DPA, HLA-DQ, HLA-DRA, TAP1, TAP2, LMP2, LMP7, RFX5, RFXAP, RFXANK, CIITA, and a combination thereof, preferably selected from HLA-A, HLA-B, HLA-C, B2M, RFX5, RFXAP, RFXANK, CIITA, and a combination thereof.

In an embodiment, the expression of at least one TCR/CD3 gene of the engineered immune cell expressing the NK inhibitory molecule is further suppressed or silenced.

T cell receptor (TCR) is a characteristic marker of the surface of all T cells. It forms a TCR/CD3 complex by binding to CD3 through a non-covalent bond, and generates a specific antigen stimulation signal by binding to a specific MHC-antigen peptide complex on the surface of the antigen presenting cell, to activate T cell and exert the killing effect. TCR is a heterodimer composed of two different peptide chains, and is generally divided into two types: α/β type and γ/δ type, in which 95% or more of peripheral T lymphocytes express TCR α/β. The TCR α chain is encoded by the TRAC gene, and the β chain is encoded by the TRBC gene. Each peptide chain of the TCR includes a variable region (V region), a constant region (C region), a transmembrane region, and a cytoplasmic region, wherein the cytoplasmic region is too short to transmit an antigen stimulation signal. TCR molecule belongs to the immunoglobulin superfamily, and the antigen specificity thereof exists in V region; the V region has three hypervariable regions, i.e. CDR1, CDR2, and CDR3, wherein the CDR3 has the maximum variation, and directly determines the antigen binding specificity of the TCR. When TCR recognizes MHC-antigen peptide complexes, CDR1 and CDR2 recognize and bind to the MHC molecule, and CDR3 directly binds to the antigen peptide. CD3 includes four subunits: γ, δ, ε, ζ, usually existing in the form of dimers εγ, εδ, ζζ. All of the four subunits comprise a conserved immunoreceptor tyrosine-based activation motif (ITAM), in which two tyrosine residues, after being phosphorylated by tyrosine protein kinases, transmit an activation signal to T cells. Thus, in an embodiment, suppressing or silencing at least one TCR/CD3 gene expression refers to suppressing or silencing the expression of one or more genes selected from the group consisting of TRAC, TRBC, CD3 γ, CD3 δ, CD3 ε, CD3 ζ.

In a preferred embodiment, the expression of at least one TCR/CD3 gene and at least one MHC-related gene in the engineered immune cell expressing the NK inhibitory molecule is suppressed or silenced, wherein the at least one TCR/CD3 gene is selected from the group consisting of TRAC, TRBC, CD3 γ, CD3 δ, CD3 ε, CD3 ζ, and a combination thereof; and the at least one MHC-related gene is selected from the group consisting of HLA-A, HLA-B, HLA-C, B2M, HLA-DPA, HLA-DQ, HLA-DRA, TAP1, TAP2, LMP2, LMP7, RFX5, RFXAP, RFXANK, CIITA, and a combination thereof, preferably selected from HLA-A, HLA-B, HLA-C, B2M, RFX5, RFXAP, RFXANK, CIITA, and a combination thereof.

In a preferred embodiment, the at least one TCR/CD3 gene is selected from the group consisting of TRAC, TRBC, and a combination thereof, and the at least one MHC-related gene is selected from the group consisting of B2M, RFX5, RFXAP, RFXANK, CIITA, and a combination thereof. In an embodiment, the expression of TRAC or TRBC and the expression of B2M of the engineered immune cell are suppressed or silenced. In an embodiment, the expression of TRAC or TRBC and the expression of CIITA of the engineered immune cell are suppressed or silenced. In a preferred embodiment, the expression of TRAC or TRBC, and the expression of B2M and CIITA of the engineered immune cell are suppressed or silenced. In a preferred embodiment, the expression of TRAC or TRBC, and the expression of B2M and RFX5 of the engineered immune cell are suppressed or silenced.

In an embodiment, in addition to the MHC-related gene and the optional TCR/CD3 gene, the expression of at least one gene selected from the following in the engineered immune cell of the present disclosure can be further suppressed or silenced: CD52, GR, dCK and immune checkpoint gene, such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3.

Methods for inhibiting gene expression or silencing a gene are well known to those skilled in the art, including but not limited to, for example, DNA breakage mediated by a meganuclease, a zincfinger nuclease, a TALE nuclease or a Cas enzyme in a CRISPR system, or inactivating a gene by antisense oligonucleotides, RNAi, shRNA, and other technologies.

### Chimeric antigen receptor

In another aspect, the engineered immune cell expressing an NK inhibitory molecule of the present disclosure further may express a chimeric antigen receptor. That is, in this embodiment, the engineered immune cell expresses an NK inhibitory molecule and a chimeric antigen receptor, and preferably, expression of at least one MHC-related gene of the engineered immune cell is suppressed or silenced. In a preferred embodiment, expression of at least one TCR/CD3 gene further included in the engineered immune cell is suppressed or silenced.

As used herein, the term "chimeric antigen receptor" or "CAR" refers to an artificially constructed hybrid polypeptide, where the hybrid polypeptide generally comprises one or more ligand binding domains (e.g., an antigen binding moiety of an antibody), a transmembrane domain, a co-stimulatory domain, and an intracellular signaling domain, and various domains are linked via a linker. CAR can redirect the specificity and reactivity of T cells and other immune cells to selected targets in a non-MHC-restricted manner by utilizing the antigen binding properties of monoclonal antibodies. Non-MHC-restricted antigen recognition gives CAR cells the ability to recognize antigen independent of antigen processing, thus bypassing the major mechanism of tumor escape. Furthermore, when expressed within T cells, CAR advantageously does not dimerize with α chain and β chain of the endogenous T cell receptor (TCR).

As used herein, "ligand binding domain" refers to any structure that can bind to a ligand (e.g. antigen) or a functional variant thereof. The ligand binding domain may be an antibody structure, including, but not limited to, monoclonal antibody, polyclonal antibody, recombinant antibody, human antibody, humanized antibody, murine antibody, chimeric antibody, and a functional fragments thereof. For example, the ligand binding domain includes, but is not limited to, intact antibody, Fab, Fab', F(ab')2, Fv fragment, scFv antibody fragment, linear antibody, sdAb (VH or VL), nanobody (Nb), recombinant fibronectin domain, anticalin, DARPIN, and so on, preferably selected from Fab, scFv, sdAb, and nanobody. In the present disclosure, the ligand binding domain may be univalent or bivalent, and may be a monospecific, bispecific or multispecific antibody.

"Fab" refers to any one of two identical fragments produced by digestion of an immunoglobulin molecule using papain, and consists of an intact light chain and a heavy chain N-terminal part linked by a disulfide bond, wherein the heavy chain N-terminal part includes a heavy chain variable region and CH1. Compared with the intact IgG, Fab has no Fc fragment, has relatively high fluidity and tissue penetration ability, and can univalently bind to an antigen without mediating antibody effects.

"Single chain antibody" or "scFv" is an antibody composed of an antibody heavy chain variable region (VH) and a light chain variable region (VL) linked by a linker. The optimal length and/or amino acid composition of the linker can be selected. The length of the linker will significantly affect the variable region folding and interaction of the scFv. In fact, intrachain folding can be prevented if a shorter linker (e.g. 5-10 amino acids) is used. Regarding the selection of size and composition of the linker, see, e.g., Hollinger et al., 1993 Proc Natl Acad. Sci. U.S.A. 90:6444-6448; US patent applications with publication Nos. 2005/0100543, 2005/0175606, 2007/0014794; and PCT applications with publication Nos. WO2006/020258 and WO2007/024715, which are incorporated herein by reference in their entirety. The scFv may contain a VH and a VL linked in any order, e.g., VH-linker-VL or VL-linker-VH.

"Single domain antibody" or "sdAb" refers to an antibody that naturally lacks a light chain, and the antibody contains only one heavy chain variable region (VHH) and two conventional CH2 and CH3 regions, also known as "heavy chain antibody".

"Nanobody" or "Nb" refers to a VHH structure that is individually cloned and expressed, which has structural stability and binding activity to an antigen comparable to those of the original heavy chain antibody, and it is the smallest unit currently known to be capable of binding to a target antigen.

The term "functional variant" or "functional fragment" refers to a variant that substantially contains the amino acid sequence of a parent, but, compared with the parent amino acid sequence, contains at least one amino acid modification (i.e., substitution, deletion or insertion), provided that the variant retains the biological activity of the parent amino acid sequence. For example, for an antibody, a functional fragment thereof is an antigen binding moiety thereof. In an embodiment, the amino acid modification is preferably a conservative modification.

As used herein, the term "conservative modification" refers to an amino acid modification that does not significantly affect or alter the binding characteristics of the antibody or antibody fragment containing the amino acid sequence. These conservative modifications include amino acid substitution, addition, and deletion. The modifications can be introduced into the chimeric antigen receptor of the present disclosure by standard techniques known in the art, such as site-directed mutagenesis and PCR-mediated mutagenesis. The conservative amino acid substitution is the substitution in which the amino acid residue is replaced with an amino acid residue having a similar side chain. Amino acid residue families having a similar side chain have been defined in the art, including basic side chain (e.g., lysine, arginine, histidine), acidic side chain (e.g., aspartic acid, glutamic acid), uncharged polar side chain (e.g., glycine, asparagine, glutamine, serine, threonine, tyrosine, cysteine), nonpolar side chain (e.g., alanine, valine, leucine, isoleucine, proline, phenylalanine, methionine, tryptophan), β-branched side chain (e.g., threonine, valine, isoleucine), and aromatic side chain (e.g., tyrosine, phenylalanine, tryptophan, histidine). The conservative modifications may be selected, for example, based on polarity, charge, solubility, hydrophobicity, hydrophilicity, and/or similarity in amphiphilic properties of residues involved.

Thus, the "functional variant" or "functional fragment" has at least 75%, preferably at least 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to the parent amino acid sequence, and retains the biological activity, e.g., binding activity, of the parent amino acid.

As used herein, the term "sequence identity" indicates the degree to which two (nucleotide or amino acid) sequences have the same residue at the same position in an alignment, and is generally expressed by percentage. Preferably, the identity is determined over the entire length of the sequences being compared. Thus, two copies with identical sequences have 100% identity. Those skilled in the art will recognize that some algorithms can be used to determine sequence identity using standard parameters, for example, Blast (Altschul et al. (1997) Nucleic Acids Res. 25:3389-3402), Blast2 (Altschul et al. (1990) J. Mol.Biol.215:403-410), Smith-Waterman (Smith et al. (1981) J. Mol.Biol.147:195-197), and ClustalW.

The selection of ligand binding domain depends on the cell surface marker on a target cell to be recognized and associated with a specific disease state, for example, a tumor specific antigen or a tumor associated antigen. Thus, in an embodiment, the ligand binding domain of the present disclosure binds to one or more targets selected from the group consisting of TSHR, CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, Folate receptor α, ERBB2 (Her2/neu), MUC1, EGFR, NCAM, Prostase, PAP, ELF2M, Ephrin B2, IGF-I receptor, CAIX, LMP2, gp100, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGF β, APRIL, Claudin18.2, NKG2D, and any combination thereof. Preferably, the target is selected from the group consisting of: CD19, CD20, CD22, BAFF-R, CD33, EGFRvIII, BCMA, GPRC5D, PSMA, ROR1, FAP, ERBB2 (Her2/neu), MUC1, EGFR, CAIX, WT1, NY-ESO-1, CD79a, CD79b, GPC3, Claudin18.2, NKG2D, and any combination thereof. Depending on the antigen to be targeted, the CAR of the present disclosure may be designed to include a ligand binding domain specific for the antigen. For example, if CD19 is the antigen to be targeted, a CD19 antibody can be used as the ligand binding domain of the present disclosure.

Structures such as transmembrane domain, co-stimulatory domain, intracellular signaling domain, and optional hinge region, signal peptide contained in the CAR that can be used in the present disclosure are defined above.

In an embodiment, the CAR of the present disclosure further may contain a switch structure, so as to regulate the expression time of the CAR. For example, the switch structure may be in the form of a dimerization domain, which causes a conformational change by binding to a corresponding ligand thereof, and exposes the extracellular binding domain to bind to a targeted antigen, thereby activating the signaling pathway. Alternatively, a switch domain also may be used to link a binding domain and a signaling domain, respectively, and only when the switch domains are bound to each other (for example, in the presence of an inducing compound), the binding domain and the signaling domain can be linked together through a dimer, thereby activating the signal pathway. The switch structure also can be in a form of a masking peptide. The masking peptide can shield the extracellular binding domain, and prevent it from binding to the targeted antigen, and after the masking peptide is cleaved by, for example, a protease, the extracellular binding domain is exposed, making it become a "normal" CAR structure. A variety of switch structures known to those skilled in the art can be used in the present disclosure.

In an embodiment, the CAR of the present disclosure further may comprise a suicide gene, i.e. making it express a cell death signal that can be induced by an exogenous substance, so as to eliminate the CAR cell when needed (e.g., when serious toxic side effects occur). For example, the suicide gene may be in the form of an inserted epitope, e.g., a CD20 epitope, an RQR8, etc., and when needed, the CAR cell can be eliminated by adding an antibody or reagent that targets these epitopes. The suicide gene also may be herpes simplex virus thymidine kinase (HSV-TK), which gene can induce cell apoptosis when receiving ganciclovir treatment. The suicide gene further may be iCaspase-9, and dimerization of iCaspase-9 can be induced by a chemical induction drug such as AP1903 and AP20187, so as to activate the downstream Caspase3 molecule, and cause apoptosis. A variety of suicide genes known to those of skill in the art can be used in the present disclosure.

When the NK inhibitory molecule contains an intracellular signaling domain and the cell expresses a CAR, the NK inhibitory molecule and CAR of the present disclosure may share structures other than the binding region, such as a co-stimulatory domain and an intracellular signaling domain, as desired. Therefore, in this embodiment, the engineered immune cell of the present disclosure: (1) expresses a fusion protein of the NK inhibitory molecule of the present disclosure and a chimeric antigen receptor, the fusion protein comprises an NK inhibitory ligand, a ligand binding domain, a transmembrane domain, a co-stimulatory domain, and an intracellular signaling domain, and (2) expression of at least one MHC-related gene is suppressed or silenced.

### Nucleic acid and vector

The present disclosure further provides a nucleic acid molecule, which contains a nucleic acid sequence encoding the NK inhibitory molecule of the present disclosure. Optionally, the nucleic acid molecule further may comprise a nucleic acid sequence encoding the chimeric antigen receptor.

As used herein, the term "nucleic acid molecule" includes a sequence of ribonucleotide and deoxyribonucleotide, such as modified or unmodified RNA or DNA, each in single-stranded and/or double-stranded form, in linear or circular or their mixtures (including hybrid molecules). Thus, the nucleic acid according to the present disclosure includes DNA (e.g. dsDNA, ssDNA, cDNA), RNA (e.g. dsRNA, ssRNA, mRNA, ivtRNA), their combinations or derivatives (e.g. PNA). Preferably, the nucleic acid is DNA or RNA, more preferably mRNA.

The nucleic acid may contain a conventional phosphodiester bond or an unconventional bond (e.g., amide bond, such as found in peptide nucleic acid (PNA)). The nucleic acid of the present disclosure further may comprise one or more modified bases, such as, for example, trityl base and uncommon base (such as inosine). Other modifications also can be contemplated, including chemical, enzymatic or metabolic modifications, so long as the multi-chain CAR of the present disclosure can be expressed from polynucleotides. The nucleic acid can be provided in isolated form. In an embodiment, the nucleic acid also may include a regulatory sequence, such as a transcriptional control element (including a promoter, an enhancer, an operon, a repressor, and a transcription termination signal), ribosome binding sites, and intron.

The nucleic acid sequences of the present disclosure can be codon-optimized for optimal expression in a desired host cell (e.g., immune cell); or for expression in a bacterial, yeast or insect cell. Codon optimization refers to substitution of a codon in the target sequence that is generally rare in highly expressed genes of a given species with a codon that is generally common in highly expressed genes of such species, and the codons before and after the substitution encode the same amino acid. Therefore, the selection of an optimal codon depends on the codon usage preference of the host genome.

The present disclosure further provides a vector, containing the nucleic acid molecule of the present disclosure. Optionally, the nucleic acid sequence encoding the NK inhibitory molecule and the nucleic acid sequence encoding the chimeric antigen receptor can be located in the same vector or different vectors.

As used herein, the term "vector" is an intermediary nucleic acid molecule used to transfer (exogenous) genetic material into a host cell, and in the host cell the nucleic acid molecule can be, for example, replicated and/or expressed.

The vector generally includes targeting vectors and expression vectors. The "targeting vector" is a medium that delivers an isolated nucleic acid to the interior of a cell by, for example, homologous recombination or by using a hybrid recombinase of a sequence at specific target site. The "expression vector" is a vector used for transcription of heterologous nucleic acid sequences (for example, those sequences encoding the chimeric antigen receptor polypeptides of the present disclosure) in suitable host cells and the translation of their mRNAs. Suitable vectors that can be used in the present disclosure are known in the art, and many are commercially available. In an embodiment, the vector of the present disclosure includes, but is not limited to, plasmid, virus (e.g., retrovirus, lentivirus, adenovirus, vaccinia virus, Rous sarcoma virus (RSV), polyoma virus, and adeno-associated virus (AAV), etc.), bacteriophage, phagemid, cosmid, and artificial chromosome (including BAC and YAC). The vector itself is usually a nucleotide sequence, and usually is a DNA sequence containing an insert (transgene) and a larger sequence as "backbone" of the vector. Engineered vector typically also contains a start siteautonomously replicating in the host cell (if stable expression of polynucleotide is desired), a selectable marker, and a restriction enzyme cleavage site (e.g., a multiple cloning site, MCS). The vectors may additionally contain elements such as a promoter, a poly-A tail (polyA), 3' UTR, an enhancer, a terminator, an insulator, an operon, a selectable marker, a reporter gene, a targeting sequence, and/or a protein purification tag. In a specific embodiment, the vector is an *in vitro* transcription vector.

### Engineered immune cell

The present disclosure further provides an engineered immune cell, which expresses the NK inhibitory molecule of the present disclosure, and wherein expression of at least one MHC-related gene is suppressed or silenced. In an embodiment, the engineered immune cell of the present disclosure further expresses a chimeric antigen receptor, wherein the chimeric antigen receptor comprises one or more ligand binding domains, a transmembrane domains, a co-stimulatory domains, and an intracellular signaling domains. In a preferred embodiment, the engineered immune cell of the present disclosure further comprises suppressed or silenced expression of at least one TCR/CD3 gene.

In an embodiment, in addition to the MHC-related gene and the optional TCR/CD3 gene, the expression of at least one gene selected from the following in the engineered immune cell of the present disclosure can be further suppressed or silenced: CD52, GR, dCK and immune checkpoint gene, such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3.

When the NK inhibitory molecule contains an intracellular signaling domain and the cell expresses a CAR, the NK inhibitory molecule and CAR of the present disclosure may share structures other than the binding region, such as the co-stimulatory domain and an intracellular signaling domain, as desired. Therefore, in this embodiment, the engineered immune cell of the present disclosure: (1) expresses a fusion protein of the NK inhibitory molecule of the present disclosure and a chimeric antigen receptor, the fusion protein comprises an NK inhibitory ligand, a ligand binding domain, a transmembrane domain, a co-stimulatory domain, and an intracellular signaling domain, and (2) expression of at least one MHC-related gene is suppressed or silenced. In a preferred embodiment, the engineered immune cell of the present disclosure further comprises suppressed or silenced expression of at least one TCR/CD3 gene.

As used herein, the term "immune cell" refers to any cell of the immune system that has one or more effector functions (e.g., cytotoxic cell killing activity, secretion of cytokines, induction of ADCC and/or CDC). For example, the immune cell may be a B cell, a T cell, a macrophage, a dendritic cell, a monocyte, an NK cell or an NKT cell, or an immune cell obtained from a stem cell source such as cell umbilical cord blood. Preferably, the immune cell is a T cell. The T cell may be any T cell, such as *in vitro* cultured T cell, for example, primary T cell or T cell from *in vitro* cultured T cell line, e.g., Jurkat, SupT1, etc. or T cell obtained from a subject. Examples of subject include humans, dogs, cats, mice, rats, and transgenic species thereof. The T cell can be obtained from a variety of sources, including peripheral blood monocytes, bone marrow, lymph node tissue, umbilical blood, thymus tissue, tissue from infection sites, ascites, pleural effusion, spleen tissue, and tumors. The T cell also may be concentrated or purified. The T cell may be at any stage of development including, but not limited to, a CD4+/CD8+ T cell, a CD4+ helper T cell (e.g., Th1 and Th2 cells), CD8+ T cell (e.g., cytotoxic T cell), tumor infiltrating cell, memory T cell, naive T cell, γδ-T cell, αβ-T cell, etc. In a preferred embodiment, the immune cell is a human T cell. The T cell can be isolated from the blood of a subject using a variety of techniques known to those of skill in the art, such as Ficoll.

The NK inhibitory molecule and optional chimeric antigen receptor can be introduced into the immune cell using conventional methods known in the art (e.g., by transduction, transfection, transformation). "Transfection" is a process of introducing a nucleic acid molecule or polynucleotide (including a vector) into a target cell. An example is RNA transfection, i.e., the process of introducing RNA (such as *in vitro* transcribed RNA, ivtRNA) into a host cell. This term is mainly used for a non-viral method in eukaryotic cells. The term "transduction" is generally used to describe virus-mediated transfer of nucleic acid molecules or polynucleotides. Transfection of animal cells typically involves opening transient pores or "holes" in the cell membrane, so as to allow uptake of material. Transfection may be carried out using calcium phosphate, by electroporation, by extrusion of cells or by mixing cationic lipids with the material so as to produce liposomes which fuse with the cell membrane and deposit their cargo into the interior. Exemplary techniques for transfecting eukaryotic host cells include lipid vesicle-mediated uptake, heat shock-mediated uptake, calcium phosphate-mediated transfection (calcium phosphate/DNA co-precipitation), microinjection, and electroporation. The term "transformation" is used to describe the non-virus transfer of a nucleic acid molecule or polynucleotide (including a vector) to bacteria, and also to non-animal eukaryotic cells (including plant cells). Thus, the transformation is a genetic alteration of bacterial or non-animal eukaryotic cells, which is produced by direct uptake of a cell membrane from its surroundings and subsequent incorporation of exogenous genetic material (nucleic acid molecule). The transformation can be achieved by artificial means. In order for transformation to occur, the cell or bacterium must be in a competent state. For prokaryotic transformation, the techniques may include heat shock-mediated uptake, fusion to bacterial protoplasts of intact cells, microinjection, and electroporation.

### Kit and pharmaceutical composition

The present disclosure provides a kit, which contains the NK inhibitory molecule, the nucleic acid molecule, the vector or the engineered immune cell of the present disclosure.

In a preferred embodiment, the kit of the present disclosure further contains instructions.

The present disclosure further provides a pharmaceutical composition, which contains the NK inhibitory molecule, the engineered immune cell, the nucleic acid molecule or the vector of the present disclosure as an active agent, and one or more pharmaceutically acceptable excipients. Therefore, the present disclosure further encompasses use of the NK inhibitory molecule, the nucleic acid molecule, the vector or the engineered immune cell in the preparation of a pharmaceutical composition or medicine.

As used herein, the term "pharmaceutically acceptable excipient" refers to a vector and/or excipient that is pharmacologically and/or physiologically compatible (i.e., capable of triggering a desired therapeutic effect without causing any undesired local or systemic effects) with the subject and active ingredient, and it is well known in the art (see, e.g., Remington's Pharmaceutical Sciences. Edited by Gennaro AR, 19th ed. Pennsylvania: Mack Publishing Company, 1995). Examples of pharmaceutically acceptable excipient include, but are not limited to, filler, binder, disintegrant, coating agent, adsorbent, anti-adherent, glidant, antioxidant, flavoring agent, colorant, sweetener, solvent, cosolvent, buffer agent, chelating agent, surfactant, diluent, wetting agent, preservative, emulsifier, cladding agent, isotonic agent, absorption delaying agent, stabilizer, and tension regulator. It is known to those skilled in the art to select a suitable excipient to prepare the desired pharmaceutical composition of the present disclosure. Exemplary excipients for use in the pharmaceutical composition of the present disclosure include saline, buffered saline, dextrose, and water. Generally, the selection of a suitable excipient depends, in particular, on the active agent used, the disease to be treated, and the desired dosage form of the pharmaceutical composition.

The pharmaceutical composition according to the present disclosure is suitable for multiple routes of administration. Generally, the administration is parenterally accomplished. Parenteral delivery methods include topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present disclosure also can be prepared in various forms, such as solid, liquid, gaseous or lyophilized forms, particularly the pharmaceutical composition can be prepared in the form of ointment, cream, transdermal patch, gel, powder, tablet, solution, aerosol, granule, pill, suspension, emulsion, capsule, syrup, elixir, extract, tincture or liquid extract, or in a form particularly suitable for the desired method of administration. Processes known in the present disclosure for producing a medicine may include, for example, conventional mixing, dissolving, granulating, dragee-making, grinding, emulsifying, encapsulating, embedding or lyophilizing process. The pharmaceutical composition containing, for example, the immune cell as described herein is generally provided in a form of solution, and preferably contains a pharmaceutically acceptable buffer.

The pharmaceutical composition according to the present disclosure further may be administered in combination with one or more other agents suitable for the treatment and/or prophylaxis of diseases to be treated. Preferred examples of agent suitable for the combination include known anti-cancer medicines such as cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, such as ricin, diphtheria toxin, pseudomonas exotoxin A, DNase and RNase; radionuclides such as iodine 131, rhenium 186, indium 111, iridium 90, bismuth 210 and 213, actinides 225 and astatine 213; prodrugs such as antibody-directed enzyme prodrugs; immunostimulatory agents such as platelet factor 4, and melanoma growth stimulating protein; antibodies or fragments thereof, such as anti-CD3 antibodies or fragments thereof, complement activators, heterologous protein domains, homologous protein domains, viral/bacterial protein domains and viral/bacterial peptides. In addition, the pharmaceutical composition of the present disclosure also can be used in combination with one or more other treatment methods, such as chemotherapy and radiotherapy.

### Therapeutic application

The present disclosure further provides a method of treating a subject with cancer, infection or autoimmune disease, including administering to the subject an effective amount of the NK inhibitory molecule, the nucleic acid molecule, the vector, the engineered immune cell or the pharmaceutical composition according to the present disclosure. Therefore, the present disclosure also encompasses use of the NK inhibitory molecule, the nucleic acid molecule, the vector, or the engineered immune cell in the preparation of a medicine for treating cancer, infection or autoimmune diseases.

In an embodiment, an effective amount of the immune cell and/or the pharmaceutical composition of the present disclosure is directly administered to the subject.

In another embodiment, the treatment method of the present disclosure is ex *vivo* treatment. Specifically, the method includes the steps of: (a) providing a sample, the sample containing an immune cell; (b) suppressing or silencing expression of at least one TCR/CD3 gene and at least one MHC-related gene of the immune cell *in vitro,* and introducing the NK inhibitory molecule of the present disclosure and an optional chimeric antigen receptor into the immune cell to obtain a modified immune cell, and (c) administering the modified immune cell to the subject in need thereof. Preferably, the immune cell provided in step (a) is selected from a B cell, a macrophage, a dendritic cell, a monocyte, a T cell, an NK cell or an NKT cell; and the immune cell can be obtained from the sample (particularly a blood sample) of the subject by conventional methods known in the art. However, other immune cells capable of expressing the chimeric antigen receptor and NK inhibitory molecule of the present disclosure and exerting the desired biological effect function as described herein also can be used. Besides, the immune cells generally selected are compatible with the subject's immune system, i.e., it is preferred that the immune cells do not trigger an immunogenic response. For example, a "general recipient cell", i.e., a universally compatible lymphocyte exerting a desired biological effect function and being capable of growing and amplifying *in vitro,* can be used. The use of such cells will not require obtaining and/or providing the subject's own lymphocyte. The ex *vivo* introduction of step (c) may be carried out by introducing the nucleic acid or vector described herein into the immune cell via electroporation or by infecting the immune cell with a viral vector, wherein the viral vector is a lentiviral vector, adenoviral vector, adeno-associated viral vector or retroviral vector as previously described. Other conceivable methods include using a transfection reagent (such as a liposome) or transient RNA transfection.

In an embodiment, the immune cell is an autologous or allogeneic cell, preferably B cell, T cell, macrophage, dendritic cell, monocyte or NK cell, NKT cell, more preferably T cell, NK cell or NKT cell.

As used herein, the term "autologous" means that any material derived from an individual will be later re-introduced into the same individual.

As used herein, the term "allogeneic" means that the material is derived from a different animal or different patient of the same species as the individual into which the material is introduced. When the genes at one or more loci are different, two or more individuals are considered allogeneic to each other. In some cases, genetic differences in allogeneic materials from various individuals of the same species may be sufficient for antigen interactions to occur.

As used herein, the term "subject" refers to a mammal. The mammal may be, but is not limited to, a human, a non-human primate, a mouse, a rat, a dog, a cat, a horse or a cow. Mammals other than human can be advantageously used as subjects representing cancer animal models. Preferably, the subject is a human.

In an embodiment, the cancer is a cancer associated with expression of the target to which the ligand binding domain binds. For example, the cancer includes, but is not limited to, brain glioma, blastoma, sarcoma, leukemia, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of digestive system, endometrial cancer, esophageal cancer, eye cancer, head and neck cancer, stomach cancer (including gastrointestinal cancer), glioblastoma (GBM), liver cancer, hepatoma, intraepithelial tumor, kidney cancer, larynx cancer, liver tumor, lung cancer (such as small cell lung cancer, non-small cell lung cancer, lung adenocarcinoma and squamous lung cancer), lymphoma (including Hodgkin's lymphoma and non-Hodgkin's lymphoma), melanoma, myeloma, neuroblastoma, oral cancer (e.g., lips, tongue, mouth, and pharynx), ovarian cancer, pancreatic cancer, prostate cancer, retinoblastoma, rhabdomyosarcoma, rectal cancer, cancer of respiratory system, salivary gland cancer, skin cancer, squamous cell carcinoma, stomach cancer, testicular cancer, thyroid cancer, uterine or endometrial cancer, malignant tumor of urinary system, vulval cancer and other cancers and sarcomas, and B cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cracked cell NHL, bulky disease NHL), mantle cell lymphoma, AIDS-related lymphoma, and Waldenstrom macroglobulinemia, chronic lymphocytic leukemia (CLL), acute lymphocytic leukemia (ALL), B cell acute lymphocytic leukemia (B-ALL), T cell acute lymphocytic leukemia (T-ALL), B cell prolymphocytic leukemia, blast cell plasmacytoid dendritic cell tumor, Burkitt lymphoma, diffuse large B cell lymphoma, follicular lymphoma, chronic myelogenous leukemia (CML), malignant lymphoproliferative disorder, MALT lymphoma, hairy cell leukemia, marginal zone lymphoma, multiple myeloma, myelodysplasia, plasmablastic lymphoma, preleukemia, plasmacytoid dendritic cell tumor, post-transplant lymphoproliferative disorder (PTLD), and other diseases associated with target expression. Preferably, the disease which can be treated with the engineered immune cell or the pharmaceutical composition of the present disclosure is selected from the group consisting of: leukemia, lymphoma, multiple myeloma, brain glioma, pancreatic cancer, stomach cancer, and so on.

In an embodiment, the infection includes, but is not limited to, infections caused by viruses, bacteria, fungi, and parasites.

In an embodiment, the autoimmune disease includes, but is not limited to, type I diabetes, celiac disease, Graves disease, inflammatory bowel disease, multiple sclerosis, psoriasis, rheumatoid arthritis, Addison disease, sicca syndrome, Hashimoto thyroiditis, myasthenia gravis, vasculitis, pernicious anemia, and systemic lupus erythematosus, etc.

In an embodiment, the method further includes administering to the subject one or more additional chemotherapeutic agents, biological agents, medicines or treatments. In this embodiment, the chemotherapeutic agents, biological agents, medicines or treatments are selected from the group consisting of radiotherapy, surgery, antibody reagent and/or small molecule and any combination thereof.

The present disclosure will be described in detail below with reference to the accompanying drawings and examples. It should be noted that those skilled in the art should understand that the accompanying drawings of the present disclosure and examples thereof are only for illustrative purpose, and cannot constitute any limitation to the present disclosure. The examples of the present disclosure and the features in the examples may be combined with each other without contradiction.

### Brief Description of Drawings

FIG. 1: Expression levels of HLA-E in E0-UNKi-T and E28-UNKi-T cells are shown.
FIG. 2: Expression levels of HLA-G in GO-UNKi-T and G28-UNKi-T cells are shown.
FIG. 3: Expression levels of E-cadherin in ECad0-UNKi-T and ECad28-UNKi-T cells are shown.
FIG. 4: Expression level of NKG2A scFv in A28-UNKi-T cells is shown.
FIG. 5: Expression level of KLRG1 in NK92-KLRG1 cells is shown.
FIG. 6: Inhibitory effect of UNKi-T cells of the present disclosure on NK cell killing effect is shown. Two-way ANOVA is used for analysis, and T test is used for statistical analysis. * indicates that P value is less than 0.05, ** indicates that P value is less than 0.01, *** indicates that P value is less than 0.001, reaching significant levels.
FIG. 7: Expression level of NKG2A scFv in A28z-UNKi-T cells is shown.
FIG. 8: Expression level of HLA-E in E28z-UNKi-T cells is shown.
FIG. 9: Killing effect of UNKi-T cells of the present disclosure on NK cells is shown. Two-way ANOVA is used for analysis, and T test is used for statistical analysis. * indicates that P value is less than 0.05, ** indicates that P value is less than 0.01, *** indicates that P value is less than 0.001, reaching significant levels.
FIG. 10: IFN-γ release levels after co-culture of UNKi-T cells of the present disclosure with NK cells are shown. Two-way ANOVA is used for analysis, and T test is used for statistical analysis. *** indicates that P value is less than 0.001, reaching significant levels.
FIG. 11: Expression level of KIR scFv in KIRG4-UNKi-T cells of the present disclosure is shown.
FIG. 12: Expression levels of LIR1 scFv in LIRG4-UNKi-1-T and LIRG4-UNKi-2-T cells of the present disclosure are shown.
FIG. 13: Inhibitory effect of UNKi-T cells of the present disclosure on NK cell killing effect is shown.
FIG. 14: Expression levels of scFv in SC7G4-T cells, SC7/SC9G4-T cells, and K1G4-T cells are shown.
FIG. 15: Inhibitory effect of SC7G4-T cells, SC7/SC9G4-T cells, and K1G4-T cells on NK cell killing effect is shown.
FIG. 16: Expression level of PDL1 in PDL1-T cells is shown.
FIG. 17: Inhibitory effect of PDL1-T cells on NK cell proliferation is shown.
FIG. 18: Expression levels of KIR scFv in NKi-B cells and NKi-Huh7 cells are shown.
FIG. 19: Inhibitory effects of NKi-B cells and NKi-Huh7 cells on NK cell killing effect are shown.

### Detailed Description of Embodiments

The T cells used in all the examples of the present disclosure are primary human CD4+CD8+ T cells isolated from healthy donors by Ficoll-PaqueTM PREMIUM (GE Healthcare, Lot No. 17-5442-02) using leukapheresis.

### Example 1: Construction of UNKi-T immune cells expressing NK inhibitory molecules and with TCR/HLA-I/HLA-II being knocked out

The following encoding sequences were synthesized, and sequentially cloned into a pGEM-T Easy vector (Promega, Lot No. A1360): B2m signal peptide (SEQ ID NO: 21), NK inhibitory ligand, CD28 hinge region (SEQ ID NO: 27), CD28 transmembrane region (SEQ ID NO: 11), wherein the NK inhibitory ligand was an extracellular region of E-cadherin (SEQ ID NO: 41, corresponding to ECad0 plasmid), a fusion molecule of B2M and HLA-E extracellular region (containing presenting peptide of SEQ ID NO: 46, B2M of SEQ ID NO: 37, and HLA-E extracellular region mutant of SEQ ID NO: 33, wherein a nucleic acid sequence of B2M was synonymously mutated SEQ ID NO: 38, corresponding to E0 plasmid), or a fusion molecule of B2M and HLA-G extracellular region (containing B2M of SEQ ID NO: 37 and HLA-G extracellular region of SEQ ID NO: 35, wherein the nucleic acid sequence of B2M was the synonymously mutated SEQ ID NO: 38, corresponding to G0 plasmid). A CD28 co-stimulatory domain (SEQ ID NO: 13) was further included in the ECad0, E0, and G0 plasmids to obtain ECad28, E28, and G28 plasmids, respectively. Correct insertion of the target sequences into the plasmids was confirmed by sequencing.

The following encoding sequences were synthesized, and sequentially cloned into pGEM-T Easy vector (Promega, Lot No. A1360): B2m signal peptide (SEQ ID NO: 21), anti-NKG2A-scFv (containing SEQ ID NOS: 5 and 7), IgG4 hinge region (SEQ ID NO: 29), CD28 transmembrane region (SEQ ID NO: 11), CD28 co-stimulatory domain (SEQ ID NO: 13), to obtain A28 plasmid, and correct insertion of the target sequence into the plasmid was confirmed by sequencing.

After 3 ml of Opti-MEM (Gibco, Lot No. 31985-070) was added to a sterile tube to dilute the above plasmids, a packaging vector psPAX2 (Addgene, Lot No. 12260) and an envelope vector pMD2.G (Addgene, Lot No. 12259) were then added according to a ratio of plasmid: virus packaging vector: virus envelope vector = 4:2:1. Then, 120 µl of X-treme GENE HP DNA transfection reagent (Roche, Lot No. 06366236001) was added, well mixed immediately, followed by incubation at room temperature for 15 min, and then the plasmid/vector/transfection reagent mixture was added dropwise into a culture flask of 293T cells. The viruses were collected at 24 and 48 hours and combined, and then subjected to ultracentrifugation (25000 g, 4°C, 2.5 hours) to obtain a concentrated lentivirus.

T cells were activated with DynaBeads CD3/CD28 CTSTM (Gibco, Lot No. 40203D), and cultured for 1 day at 37°C and 5% CO₂. Then, the concentrated lentivirus was added, and after 3 days of continuous culture, T cells expressing NK inhibitory molecule were obtained.

TCR/CD3 component (specifically TRAC gene) and MHC-related genes (specifically B2M and RFX5) in the T cells expressing the NK inhibitory molecule were then knocked out using the CRISPR system. Specifically, 10 µg of Cas9 protein and 10 µg of sgRNA (3.3 µg TRAC sgRNA (SEQ ID NO: 43) +3.3 µg of B2m sgRNA (SEQ ID NO: 44) +3.3 µg of RFX5 sgRNA (SEQ ID NO: 45)) were electrically transfected into activated NKi-T cells at 400 V, 0.7 ms using a BTX Agile Pulse Max electroporator (Harvard Apparatus BTX). Immediately after electrotransfection, the NKi-T cells were placed in 1 ml of preheated medium, and cultured in the presence of IL-2 (300 IU/ml) at 37°C and 5% CO₂ to obtain TCR/B2M/RFX5 triple knockout UNKi-T cells. The wild-type T cell with TCR/B2M/RFX5 being knocked out by the CRISPR system (i.e., Mock T cell) and the wild-type T cell without gene knockout (i.e., NT cell) were used as control.

The structure of the NK inhibitory molecule contained in the UNKi-T cells prepared in the present example is as shown in Table 1 below.

**Table 1. UNKi-T cell comprising NK inhibitory molecule**

| Cell Name | signal peptide | NK inhibitory ligand | hinge region | transmembrane domain | co-stimulatory domain |
|---|---|---|---|---|---|
| Ecad0-UNKi-T | B2M | E-cadherin extracellular regio n | CD28 | CD28 | none |
| Ecad28-UNKi-T | B2M | E-cadherin extracellular region | CD29 | CD28 | CD28 |
| E0-UNKi-T | B2M | presenting peptide + synonymously mutated B2M+ HLA-E extracellular region mutant | none | CD28 | none |
| E28-UNKi-T | B2M | presenting peptide + synonymously mutated B2M+ HLA-E extracellular region mutant | none | CD28 | CD28 |
| GO-UNKi-T | B2M | synonymously mutated B2M HLA-G extracellular region | none | CD28 | none |
| G28-UNKi-T | B2M | synonymously mutated B2M+ HLA-G extracellular region | none | CD28 | CD28 |
| A28-UNKi-T | B2M | anti-NKG2A-scFv | IgG4 | CD28 | CD28 |

After 11 days, expression efficiencies of TCR/HLA-I/HLA-II in UNKi-T cell, Mock T cell, and NT cell were measured by a flow cytometer using FITC Mouse Anti-Human CD3 (BD Pharmingen, Lot No. 555916) antibody, PE mouse anti-human HLA-I (R&D, Lot No. FAB7098P) and APC anti-human DR, DP, DQ (biolegend, Lot No. 361714) antibody, and results are shown in Table 2 below.

**Table 2. Gene expression efficiency in UNKi-T cell**

| Cell Name | TCR/CD3 | B2M/HLA-I | RFX5/HLA-II |
|---|---|---|---|
| Ecad0-UNKi-T | 3.6% | 19% | 11% |
| Ecad28-UNKi-T | 2.9% | 18.3% | 10.4% |
| E0-UNKi-T | 4.8% | 18.9% | 11.3% |
| E28-UNKi-T | 3.4% | 20% | 12% |
| GO-UNKi-T | 4% | 19% | 9.8% |
| G28-UNKi-T | 2.9% | 19.7% | 10% |
| A28-UNKi-T | 3.8% | 21.1% | 12.1% |
| Mock T | 2.7% | 17.8% | 9.8% |
| NT | 97% | 98% | 60% |

As can be seen from Table 2, expression of TCR/B2M/RFX5 in UNKi-T cell prepared in the present disclosure and Mock T cell is effectively suppressed or silenced.

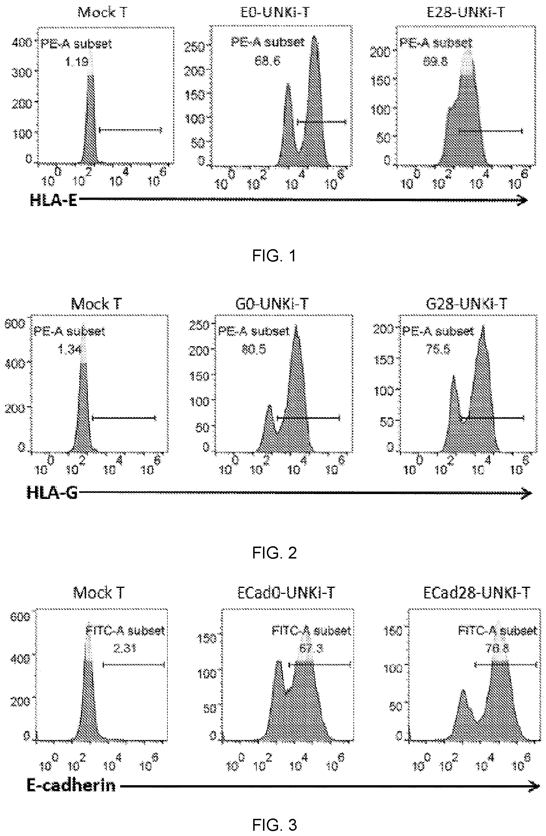

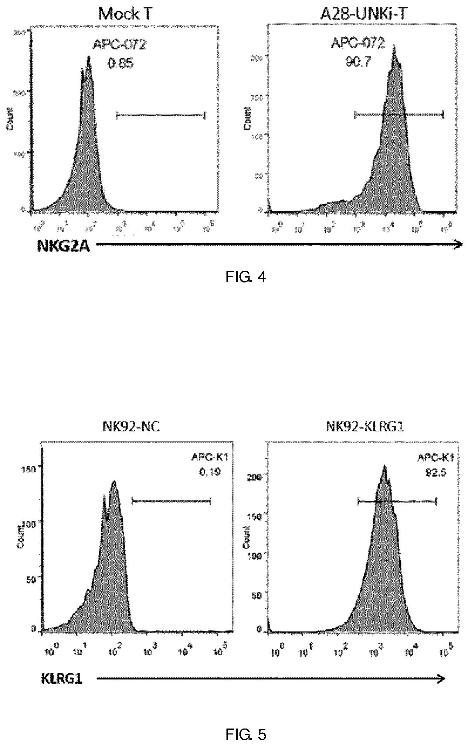

Furthermore, HLA-E expression in UNKi-T cell and Mock T cell was detected by a flow cytometer using PE mouse anti-human HLA-E (biolegend, Lot No. 342604) (FIG. 1), HLA-G expression in UNKi-T cell and Mock T cell was detected using PE mouse anti-human HLA-G (biolegend, Lot No. 335906) (FIG. 2), E-cadherin expression in UNKi-T cell and Mock T cell was detected using E-cadherin monoclonal antibody (invitrogen, Lot No. 13-5700) and Goat anti-Mouse IgG (H+L) Cross-Adsorbed Secondary Antibody, Alexa Fluor 488 (Invitrogen, Lot No. A-11001) (FIG. 3), and anti-NKG2A scFv expression in UNKi-T cell and Mock T cell was detected using Biotin-SP (long spacer) AffiniPure Goat Anti-Human IgG, F(ab') fragment specific antibody (Jackson ImmunoResearch, Lot No. 109-065-097) and APC Streptavidin (BD, Lot No. 554067) (FIG. 4).

It can be seen from FIGS. 1-4 that the NK inhibitory molecules in the UNKi-T cell prepared in the present disclosure are all effectively expressed.

### Example 2. Inhibitory effect of UNKi-T cell on NK cell killing effect

The effector cells used in the present example are NK92 cells. Because the NK92 cell line does not express the receptor KLRG1 of E-cadherin, NK92 cell overexpressing KLRG1 was prepared first.

Nucleic acid sequences encoding KLRG1 (SEQ ID NO: 54) were synthesized, and sequentially cloned into pGEM-T Easy vector (Promega, Lot No. A1360), and the correct insertion of the target sequence was confirmed by sequencing. The vector was subjected to enzyme digestion with Spel enzyme, purified, and recovered to obtain a linearized vector. Then mRNA was prepared using mMESSAGE mMACHINE@T7 Ultra Kit (Invitrogen, Lot No. AM1345) with a linearized vector as a template according to the manufacturer's recommendations, and purified with Fastpure cell/Tissue total RNA isolation kit (Vazyme, Lot No. RC101-01) to obtain purified mRNA. Then 20 µg of purified mRNA prepared above was electrically transfected into NK92 cells at 200 V and 2 ms using a BTX Agile Pulse Max electroporator (Harvard Apparatus BTX) to obtain NK92-KLRG1 cells. 16 h later, the expression of KLRG1 was detected, and result is as shown in FIG. 5. NK92 cells not transfected with KLRG1 served as control.

As can be seen from FIG. 5, NK92-KLRG1 cells can effectively express KLRG1.

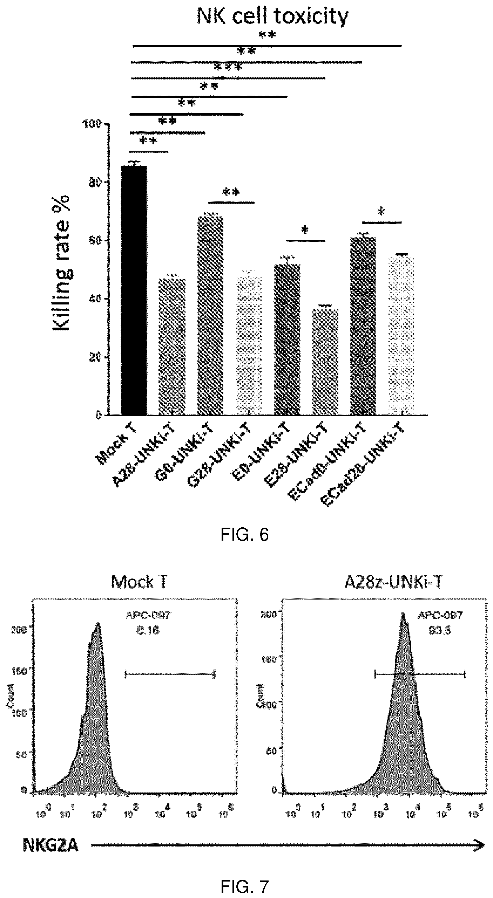

The inhibitory effect of UNKi-T cells prepared in the present disclosure on NK cell killing effect was then detected according to the following method: UNKi-T cells prepared in the present disclosure and Mock-T cells were labeled with Far-Red (invitrogen, Lot No. C34564). Labeled UNKi-T cells and Mock T cells were then plated into a 96-well plate at a concentration of 1×10⁴ cells per well, and were co-cultured with NK92 cells (for Mock T cells and UNKi-T cells expressing HLA-E, HLA-G or NKG2A scFv) or NK92-KLRG1 cells (for UNKi-T cells expressing E-cadherin) at an effector-target ratio of 2:1. After 16-18 hours, the ratio of T cells in the culture was detected by a flow cytometer, and the killing rate of the NK cells to the T cells was further calculated, and the results are shown in FIG. 6.

It can be seen from FIG. 6 that compared with Mock T cells not expressing NK inhibitory molecules, UNKi-T cells expressing NK inhibitory molecules containing inhibitory ligands such as NKG2A scFv, HLA-G, HLA-E, and E-cadherin can significantly reduce the killing effect of NK cells on T cells. Moreover, compared with T cells only expressing inhibitory ligands and transmembrane domain, the addition of co-stimulatory domain may further significantly enhance inhibition of NK cell killing against T cells (see G0 vs G28; E0 vs E28; ECad0 vs ECad28). Therefore, the NK inhibitory molecule comprising an inhibitory ligand,a transmembrane domain, and a co-stimulatory domain prepared in the present disclosure can significantly reduce the killing effect of NK cells on UNKi-T cells, thereby being capable of effectively reducing the risk of HvGD.

### Example 3. Killing effect of UNKi-T cell on NK cell

E28z-UNKi-T and A28z-UNKi-T cells were prepared according to the method in Example 1, which differ from E28-UNKi-T and A28-UNKi-T cells only in that they further comprise a CD3 ζ intracellular signaling domain (SEQ ID NO: 17).

### (1) Detecting expression of NK inhibitory molecule

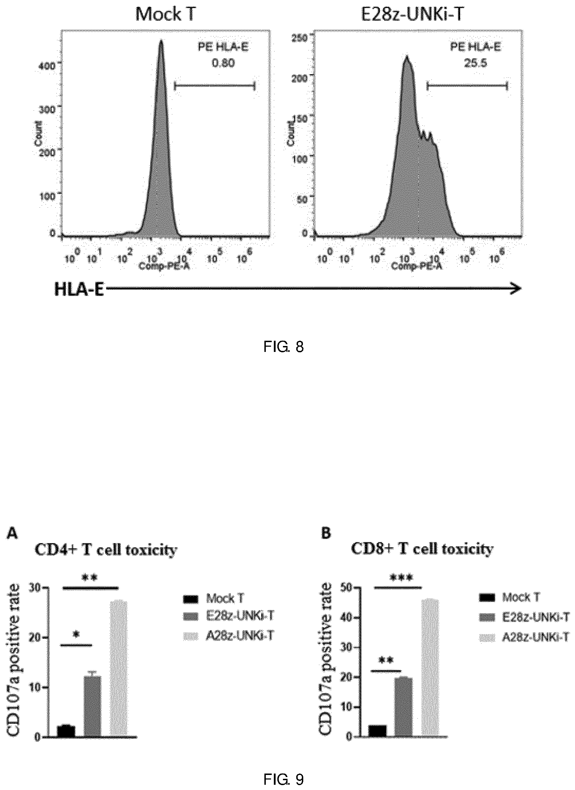

The anti-NKG2A scFv expression in A28z-UNKi-T cells and Mock T cells was detected with Biotin-SP (long spacer) AffiniPure Goat Anti-Human IgG, F(ab') fragment specific antibody (Jackson ImmunoResearch, Lot No. 109-065-097) and APC Streptavidin (BD, Lot No. 554067) (FIG. 7). The HLA-E expression in E28z-UNKi-T cells and Mock T cells was detected with PE mouse anti-human HLA-E (biolegend Lot No. 342604) by a flow cytometer (FIG. 8).

It can be seen that the NK inhibitory molecules in the E28z-UNKi-T cells and A28z-UNKi-T cells prepared in the present disclosure can be effectively expressed.

### (2) Detecting expression of CD107a

The cytotoxic T lymphocyte (CTL cell) cytoplasm contains a high concentration of cytotoxic particles in the form of vesicles, and a lysosome associated membrane protein I (CD107a) is a main component of the vesicle membrane protein. When the CTL cell kills the target cell, toxic particles will reach the cell membrane and fuse with it (at this time, the CD107a molecule is transported to the surface of the cell membrane), causing release of particle content, and finally death of the target cell. Therefore, CD107a molecule is a sensitive marker for degranulation of CTL cells, and can reflect the cell killing activity.

Target cells (NK92 cells) were plated in a 96-well plate at a concentration of 1×10⁵ cells/well, then Mock T cells, E28z-UNKi-T cells, and A28z-UNKi-T cells were added at a ratio of 1:1 per well, meanwhile 10 µl of PE-anti-human CD107a (BD Pharmingen, Lot No. 555801) was added for co-culture at 37°C and 5% CO₂ culture condition. After 1 h, Goigstop (BD Pharmingen, Lot No. 51-2092KZ) was added for further incubation of 2.5 h. 5 µl of APC-anti human CD8 (BD Pharmingen, Lot No.: 555369) and 5 µl of FITC-antihuman CD4 (BD Pharmingen, Lot No.: 561005) were then added to each well, after 30 minutes of incubation at 37°C, the expression level of CD107a was detected by a flow cytometer, and results are as shown in FIG. 9A (CD4+ T cell toxicity) and FIG. 9B (CD8+ T cell toxicity).

It can be seen that Mock T cells not expressing NK inhibitory molecules seldom kill target cells. On the contrary, after E28z-UNKi-T cells and A28z-UNKi-T cells prepared in the present disclosure were co-cultured with target cells, the expression rate of CD107a was significantly improved, indicating that the UNKi-T cells of the present disclosure can significantly kill the NK cells.

### (3) Detecting IFN-y secretion

Target cells (NK92 cells) were plated in a 96-well plate at 1×10⁵ cells/well, Mock T cells, E28z-UNKi-T cells, and A28z-UNKi-T cells were added at a ratio of 1:1 per well, and co-cultured at 37°C and 5% CO₂ culture condition. After 18-24 hours, cell co-culture supernatant was collected.

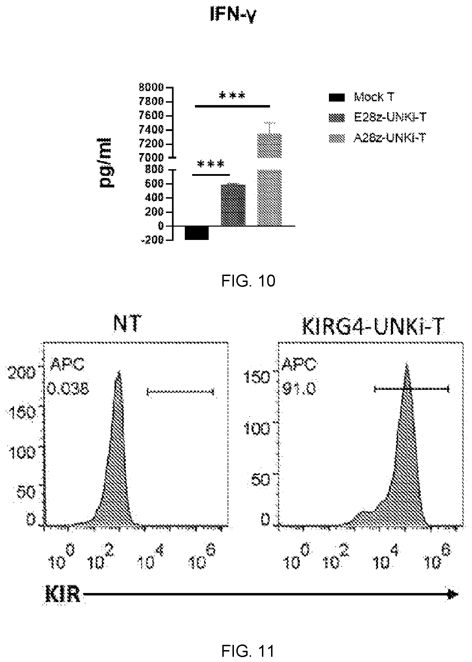

The 96-well plate was coated with capture antibody Purified anti-human IFN-γ Antibody (Biolegend, Lot No. 506502) and incubated overnight at 4°C, then the antibody solution was removed. 250 µL of PBST (1X PBS containing 0.1% Tween) solution containing 2% BSA (sigma, Lot No. V90093-1kg) was added, and incubated at 37°C for 2 hours. The plate was then washed 3 times with 250 µL of PBST (1XPBS containing 0.1% Tween). 50 µL of cell co-culture supernatant or a standard was added to each well, followed by incubation at 37°C for 1 hour, then the plate was washed 3 times with 250 µL of PBST (1XPBS containing 0.1% Tween). Then 50 µL of detection antibody Anti-Interferon gamma antibody [MD-1] (Biotin) (abcam, Lot No. ab25017) was added to each well, and after 1 hour of incubation at 37°C, the plate was washed 3 times with 250 µL of PBST (1XPBS containing 0.1% Tween). HRP Streptavidin (Biolegend, Lot No. 405210) was then added, and after 30 minutes of incubation at 37°C, the supernatant was discarded, 250 µL of PBST (1XPBS containing 0.1% Tween) was added, and the plate was washed 5 times. 50 µL of TMB substrate solution was added to each well. The reaction was carried out in the dark at room temperature for 30 minutes, after which 50 µL of 1 mol/L H₂SO₄ was added to each well to stop the reaction. Within 30 minutes after the stop of the reaction, the absorbance at 450 nm was detected by a plate reader, and the content of cytokines was calculated according to a standard curve (drawn according to read value and concentration of the standard), and the result is as shown in FIG. 10.

It can be seen that cytokine IFN-y release levels of E28z-UNKi-T cell and A28z-UNKi-T cell of the present disclosure are much higher than that of Mock T cell, which also indicates that their killing against the NK92 target cell is significantly increased.

### Example 4. UNKi-T cell targeting KIR or LIR1 and inhibitory effect thereof on NK cell killing effect

The following encoding sequences were synthesized, and sequentially cloned into the pLVX vector (Public Protein/Plasmid Library (PPL), Lot No.: PPL00157-4a): B2m signal peptide (SEQ ID NO: 21), anti-KIR-scFv (containing SEQ ID NOS: 55 and 56) or anti-LIR1 scFv (containing SEQ ID NOS: 57 and 58, or containing SEQ ID NOS: 59 and 60), IgG4 hinge region (SEQ ID NO: 29), CD8 α transmembrane region (SEQ ID NO: 9), CD28 co-stimulatory domain (SEQ ID NO: 13), to obtain KIRG4, LIRG4-1, and LIRG4-2 plasmids, and the correct insertion of the target sequence into the plasmids was confirmed by sequencing.

UNKi-T cells were prepared according to knockout and infection method in Example 1, and expression efficiencies of CD3/HLA-I/HLA-II in UNKi-T cell, Mock T cell, and NT cell were measured by a flow cytometer using FITC Mouse Anti-Human CD3 (BD Pharmingen, Lot No. 555916) antibody, PE mouse anti-human HLA-I (R&D, Lot No. FAB7098P) and APC anti-human DR, DP, DQ (biolegend, Lot No. 361714) antibody, and results are shown in Table 3 below.

**Table 3. Gene expression efficiency in UNKi-T cell**

| Cell Name | TCR/CD3 | B2M/HLA-I | RFX5/HLA-II |
|---|---|---|---|
| KIRG4-UNKi-T | 4.5% | 16% | 10.8% |
| LIRG4-UNKi-1-T | 3.5% | 17.6% | 10.9% |
| LIRG4-UNKi-2-T | 4.3 % | 16.9% | 10.3% |
| Mock T | 2.6% | 15.8% | 9.3% |
| NT | 98% | 98% | 83% |

As can be seen from Table 3, expression of CD3/HLA-I/HLA-II in UNKi-T cell prepared in the present disclosure and Mock T cell is effectively suppressed or silenced.

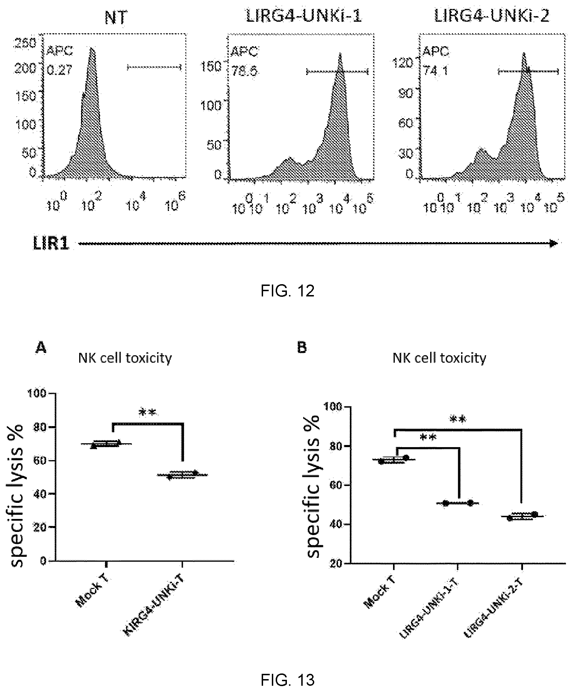

The scFv expression in KIRG4-UNKi-T cell was detected with Biotin-SP (long spacer) AffiniPure Goat Anti-Human IgG, F(ab') fragment specific antibody (Jackson ImmunoResearch, Lot No. 109-065-097) and APC Streptavidin (BD, Lot No. 554067), the results are as shown in FIG. 11. The scFv expression in LIRG4-UNKi-1 T cell and LIRG4-UNKi-2 T cell was detected with recombinant human LILRB1 protein (sino biological, Lot No. 16014-H02H) as primary antibody, APC anti-human IgG Fc (biolegend, Lot No. 409306) as secondary antibody, the results are as shown in FIG. 12. It can be seen from FIG. 11 and FIG. 12 that the scFvs of the UNKi-T cell prepared in the present disclosure are all effectively expressed.

UNKi-T cell and NK92 cell were co-cultured according to the method of Example 2 to detect the inhibitory effect of UNKi-T cell on NK cell killing effect, and the results are as shown in FIG. 13. It can be seen that compared with Mock T, all the UNKi-T cells targeting KIR (FIG. 13A) or LIR1 (FIG. 13B) prepared in the present example can significantly reduce the killing effect of NK cells on T cells.

### Example 5. T cell targeting SIGLEC7, SIGLEC9 or KLRG1 and inhibitory effect thereof on NK cell killing effect

The following encoding sequences were synthesized, and sequentially cloned into the pLVX vector (Public Protein/Plasmid Library (PPL), Lot No.: PPL00157-4a): B2m signal peptide (SEQ ID NO: 21), anti-SIGLEC7-scFv (SEQ ID NO: 130), anti-SIGLEC7/SIGLEC9-scFv (SEQ ID NO: 184), or anti-KLRG1-scFv (SEQ ID NO: 119), IgG4 hinge region (SEQ ID NO: 29), CD8 α transmembrane region (SEQ ID NO: 9), CD28 co-stimulatory domain (SEQ ID NO: 13), to obtain SC7G4, SC7/SC9G4, and K1G4 plasmids, and the correct insertion of the target sequence into the plasmids was confirmed by sequencing.

The above plasmids were transferred into T cells according to the method in Example 1, and the B2M gene therein was knocked out to obtain NKi-T cells (namely, SC7G4-T cells, SC7/SC9G4-T cells, and K1G4-T cells) expressing NK inhibitory molecules and with B2M being knocked out. T cells with only B2M being knocked out served as negative control (NT).

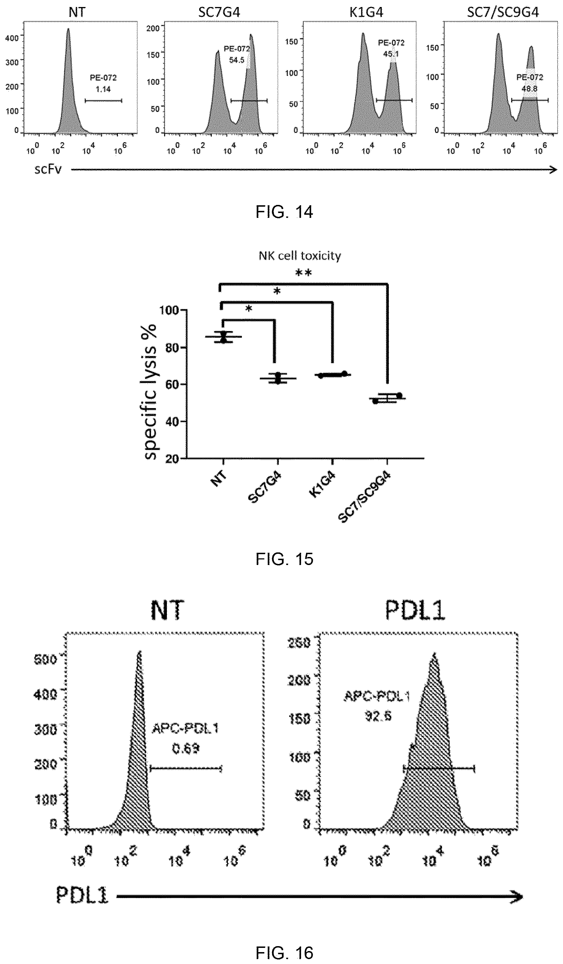

The scFv expression in SC7G4, SC7/SC9G4, and K1G4T cell was detected with Biotin-SP (long spacer) AffiniPure Goat Anti-mouse IgG, F(ab') fragment specific antibody (Jackson ImmunoResearch, Lot No. 115-066-072) and APC Streptavidin (BD, Lot No. 554067), the results are as shown in FIG. 14. It can be seen that the scFvs of the NKi-T cells prepared in the present disclosure are all effectively expressed.

According to the method of Example 2, the above NKi-T cell was co-cultured with NK92-KLRG1 cell to detect its inhibitory effect on the killing effect of NK cell, and results are as shown in FIG. 15. It can be seen that compared with NT, all the T cells targeting SIGLEC7, SIGLEC9 or KLRG1 prepared in the present example can significantly reduce the killing effect of NK cell on T cell.

### Example 6. T cell targeting PD-1 and inhibitory effect thereof on NK cell proliferation

The following encoding sequences were synthesized, and sequentially cloned into a pLVX vector (Public Protein/Plasmid Library (PPL), Lot No.: PPL00157-4a): PDL1 signal peptide (SEQ ID NO: 121), PDL1 extracellular region (SEQ ID NO: 70), PDL1 transmembrane region (SEQ ID NO: 120), CD28 co-stimulatory domain (SEQ ID NO: 13) to obtain a PDL1 plasmid, and correct insertion of the target sequence into the plasmid was confirmed by sequencing.

The above plasmid was transferred into T cell according to the method in Example 1 to obtain NKi-T cell expressing NK inhibitory molecule, namely, PDL1-T cell. Expression thereof was detected with Anti-PD-L1 Antibody (manufacturer: Solarbio, Lot No.: 10084-R312-A), and results are as shown in FIG. 16. It can be seen that PDL1 is effectively expressed. Untreated T cell served as negative control (NT).

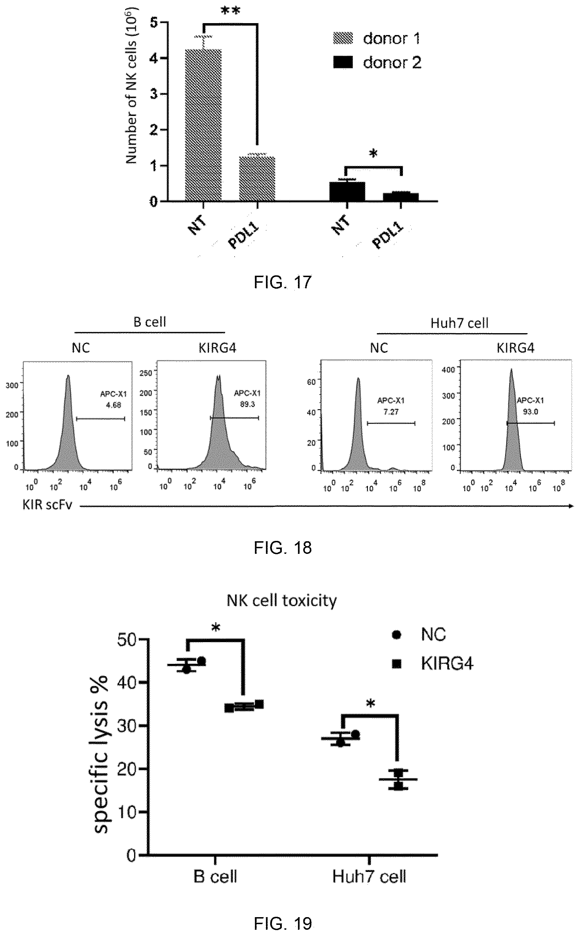

The above NKi-T cell was cultured and treated with cell mitomycin C. Two kinds of PBMCs from foreign sources (donor 1 and donor 2) were labeled with Far-Red, and then were co-cultured with T cell at a ratio of T cell: PBMC =1:2. Half-medium-replacement treatment was performed every 2-3 days. After 8 days, cells were counted and stained with PE anti-human CD3 (manufacturer biolegend, Lot No.: 317308) and FITC anti-human CD56 (manufacturer: biolegend, Lot No.: 362546), and then NK cell population ratio was detected by flow cytometry. The number of NK cells was calculated by the total cell amount * NK cell population ratio, and results are as shown in FIG. 17. It can be seen that NKi-T cell targeting PD-1 can significantly inhibit the proliferation of NK cell.

### Example 7. Inhibitory effect of B cell and Huh7 cell targeting KIR on NK cell killing effect

The KIRG4 plasmid prepared in Example 4 was transductedc into B cell and Huh7 cell (liver cancer cell) according to the method in Example 1, and the B2M gene therein was knocked out to obtain NKi-B cell and NKi-Huh7 cell expressing NK inhibitory molecule and with B2M being knocked out. B cell and Huh7 cell (NC cell), with only B2M gene being knocked out, served as negative control.

The scFv expression in NKi-B cell and NKi-Huh7 cell was detected with Biotin-SP (long spacer) AffiniPure Goat Anti-Human IgG, F(ab') fragment specific antibody (Jackson ImmunoResearch, Lot No. 109-065-097) and APC Streptavidin (BD, Lot No. 554067), and results are as shown in FIG. 18. It can be seen that the NK inhibitory molecules are effectively expressed in both B cell and Huh7 cell.

The cells prepared in the above and NK cells were co-cultured according to the method of Example 2 to detect the inhibitory effect of the above cell on the killing effect of NK cell, and results are as shown in FIG. 19. It can be seen that compared with the NC cell, both NKi-B cell and NKi-Huh7 cell prepared in the present example can significantly reduce the killing effect of NK cell on them.

It should be noted that the above-mentioned are merely for preferred examples of the present disclosure and not used to limit the present disclosure. For one skilled in the art, various modifications and changes may be made to the present disclosure. Those skilled in the art should understand that any amendments, equivalent replacements, improvements, and so on, made within the spirit and principle of the present disclosure should be covered within the scope of protection of the present disclosure.

## Claims

1. An NK inhibitory molecule, **characterized by** comprising one or more NK inhibitory ligands, a transmembrane domain, and a co-stimulatory domain, wherein the one or more NK inhibitory ligands specifically bind to NK inhibitory receptors (NKIR) to inhibit killing of NK cells against an engineered immune cell expressing the NK inhibitory molecule.

2. The NK inhibitory molecule according to claim 1, wherein the NK inhibitory ligand is an antibody targeting NKIR, ora natural ligand of NKIR or an NKIR binding region contained therein.

3. The NK inhibitory molecule according to claim 1 or 2, wherein the NKIR is selected from the group consisting of an NKG2/CD94 component, a killer cell Ig-like receptor (KIR) family member, a leukocyte Ig-like receptor (LIR) family member, an NK cell receptor protein 1 (NKR-P1) family member, an immune checkpoint receptor, a carcinoembryonic antigen-related cellular adhesion molecule 1 (CEACAM1), a sialic acid-binding immunoglobulin-like lectin (SIGLEC) family member, an leukocyte-associated immunoglobulin-like receptor 1 (LAIR1), an Ly49 family member and a killer cell lectin-like receptor G1 (KLRG1).

4. The NK inhibitory molecule according to claim 3, wherein the NKG2/CD94 component is selected from the group consisting of NKG2A, NKG2B, and CD94; the KIR family member is selected from the group consisting of KIR2DL1, KIR2DL2/3, KIR2DL5A, KIR2DL5B, KIR3DL1, KIR3DL2, and KIR3DL3; the LIR family member is selected from the group consisting of LIR1, LIR2, LIR3, LIR5, and LIR8; the NKR-P1 family member is selected from the group consisting of NKR-P1B and NKR-P1D; the immune checkpoint receptor is selected from the group consisting of PD-1, TIGIT, CD96, TIM3, and LAG3; the SIGLEC family member is selected from the group consisting of SIGLEC7 and SIGLEC9; and the Ly49 family member is selected from the group consisting of Ly49A, Ly49C, Ly49F, Ly49G1, and Ly49G4.

5. The NK inhibitory molecule according to claim 3 or 4, wherein the NKIR is selected from the group consisting of NKG2A, NKG2B, CD94, LIR1, LIR2, LIR3, KIR2DL1, KIR2DL2/3, KIR3DL1, CEACAM1, PD1, LAIR1, SIGLEC7, SIGLEC9, and KLRG1.

6. The NK inhibitory molecule according to any one of claims 1-5, wherein the NK inhibitory ligand is an antibody targeting NKIR or a functional fragment thereof, and the antibody or the functional fragment thereof is selected from the group consisting of an intact antibody, Fab, Fab', F(ab')2, an Fv fragment, an scFv antibody fragment, a linear antibody, sdAb or a nanobody.

7. The NK inhibitory molecule according to any one of claims 1-5, wherein the NK inhibitory ligand is an antibody targeting PD1, NKG2A, LIR1, KIR, SIGLEC7, SIGLEC9 and/or KLRG1.

8. The NK inhibitory molecule according to claim 7, wherein
(i) the antibody targeting NKG2A comprises (1) CDR-L1 as represented by SEQ ID NO: 72, CDR-L2 as represented by SEQ ID NO: 73, CDR-L3 as represented by SEQ ID NO: 74, CDR-H1 as represented by SEQ ID NO: 75, CDR-H2 as represented by SEQ ID NO: 76, and CDR-H3 as represented by SEQ ID NO: 77, or (2) CDR-L1 as represented by SEQ ID NO: 78, CDR-L2 as represented by SEQ ID NO: 79, CDR-L3 as represented by SEQ ID NO: 80, CDR-H1 as represented by SEQ ID NO: 81, CDR-H2 as represented by SEQ ID NO: 82, and CDR-H3 as represented by SEQ ID NO: 83;
(ii) the antibody targeting LIR1 comprises (1) CDR-L1 as represented by SEQ ID NO: 90, CDR-L2 as represented by SEQ ID NO: 91, CDR-L3 as represented by SEQ ID NO: 92, CDR-H1 as represented by SEQ ID NO: 93, CDR-H2 as represented by SEQ ID NO: 94, and CDR-H3 as represented by SEQ ID NO: 95, or (2) CDR-L1 as represented by SEQ ID NO: 96, CDR-L2 as represented by SEQ ID NO: 97, CDR-L3 as represented by SEQ ID NO: 98, CDR-H1 as represented by SEQ ID NO: 99, CDR-H2 as represented by SEQ ID NO: 100, and CDR-H3 as represented by SEQ ID NO: 101;
(iii) the antibody targeting KIR comprises CDR-L1 as represented by SEQ ID NO: 84, CDR-L2 as represented by SEQ ID NO: 85, CDR-L3 as represented by SEQ ID NO: 86, CDR-H1 as represented by SEQ ID NO: 87, CDR-H2 as represented by SEQ ID NO: 88, and CDR-H3 as represented by SEQ ID NO: 89;
(iv) the antibody targeting SIGLEC7, SIGLEC9 or both comprises (1) CDR-L1 as represented by SEQ ID NO: 102, CDR-L2 as represented by SEQ ID NO: 103, CDR-L3 as represented by SEQ ID NO: 104, CDR-H1 as represented by SEQ ID NO: 105, CDR-H2 as represented by SEQ ID NO: 106, and CDR-H3 as represented by SEQ ID NO: 107, (2) CDR-L1 as represented by SEQ ID NO: 122, CDR-L2 as represented by SEQ ID NO: 123, CDR-L3 as represented by SEQ ID NO: 124, CDR-H1 as represented by SEQ ID NO: 125, CDR-H2 as represented by SEQ ID NO: 126 and CDR-H3 as represented by SEQ ID NO: 127, (3) CDR-L1 as represented by SEQ ID NO: 131, CDR-L2 as represented by SEQ ID NO: 132, CDR-L3 as represented by SEQ ID NO: 133, CDR-H1 as represented by SEQ ID NO: 134, CDR-H2 as represented by SEQ ID NO: 135 and CDR-H3 as represented by SEQ ID NO: 136, (4) CDR-L1 as represented by SEQ ID NO: 140, CDR-L2 as represented by SEQ ID NO: 141, CDR-L3 as represented by SEQ ID NO: 142, CDR-H1 as represented by SEQ ID NO: 143, CDR-H2 as represented by SEQ ID NO: 144, and CDR-H3 as represented by SEQ ID NO: 155, (5) CDR-L1 as represented by SEQ ID NO: 176, CDR-L2 as represented by SEQ ID NO: 177, CDR-L3 as represented by SEQ ID NO: 178, CDR-H1 as represented by SEQ ID NO: 179, CDR-H2 as represented by SEQ ID NO: 180 and CDR-H3 as represented by SEQ ID NO: 181, or (6) CDR-L1 as represented by SEQ ID NO: 188, CDR-L2 as represented by SEQ ID NO: 189, CDR-L3 as represented by SEQ ID NO: 190, CDR-H1 as represented by SEQ ID NO: 191, CDR-H2 as represented by SEQ ID NO: 192, and CDR-H3 as represented by SEQ ID NO: 193; and/or
(v) the antibody targeting KLRG1 comprises (1) CDR-L1 as represented by SEQ ID NO: 111, CDR-L2 as represented by SEQ ID NO: 112, CDR-L3 as represented by SEQ ID NO: 113, CDR-H1 as represented by SEQ ID NO: 114, CDR-H2 as represented by SEQ ID NO: 115, and CDR-H3 as represented by SEQ ID NO: 116, (2) CDR-L1 as represented by SEQ ID NO: 149, CDR-L2 as represented by SEQ ID NO: 150, CDR-L3 as represented by SEQ ID NO: 151, CDR-H1 as represented by SEQ ID NO: 152, CDR-H2 as represented by SEQ ID NO: 153 and CDR-H3 as represented by SEQ ID NO: 154, (3) CDR-L1 as represented by SEQ ID NO: 158, CDR-L2 as represented by SEQ ID NO: 159, CDR-L3 as represented by SEQ ID NO: 160, CDR-H1 as represented by SEQ ID NO: 161, CDR-H2 as represented by SEQ ID NO: 162, and CDR-H3 as represented by SEQ ID NO: 163, or (4) CDR-L1 as represented by SEQ ID NO: 167, CDR-L2 as represented by SEQ ID NO: 168, CDR-L3 as represented by SEQ ID NO: 169, CDR-H1 as represented by SEQ ID NO: 170, CDR-H2 as represented by SEQ ID NO: 171, and CDR-H3 as represented by SEQ ID NO: 172.

9. The NK inhibitory molecule according to any one of claims 1-5, wherein the NK inhibitory ligand is selected from the group consisting of HLA-E, HLA-F, HLA-G, cadherin, collagen, OCIL, sialic acid, PD-L1/PD-L2, CD155, CD112, CD113, Gal-9, FGL1, and an NKIR binding region contained therein.

10. The NK inhibitory molecule according to claim 9, wherein the NK inhibitory ligand is selected from the group consisting of sialic acid, HLA-E extracellular region, HLA-F extracellular region, HLA-G extracellular region, E-cadherin extracellular region, PD-L1 extracellular region, and PD-L2 extracellular region.

11. The NK inhibitory molecule according to claim 10, wherein
(i) the HLA-E extracellular region has at least 70%, preferably at least 80%, and more preferably at least 90%, 95%, 97% or 99% or 100% sequence identity to an amino acid sequence represented by SEQ ID NO: 31 or 33;
(ii) the HLA-G extracellular region has at least 70%, preferably at least 80%, and more preferably at least 90%, 95%, 97% or 99% or 100% sequence identity to an amino acid sequence represented by SEQ ID NO: 35;
(iii) the E-cadherin extracellular region has at least 70%, preferably at least 80%, and more preferably at least 90%, 95%, 97% or 99% or 100% sequence identity to an amino acid sequence represented by SEQ ID NO: 39 or 41;
(iv) the PD-L1 extracellular region has at least 70%, preferably at least 80%, and more preferably at least 90%, 95%, 97% or 99% or 100% sequence identity to an amino acid sequence represented by SEQ ID NO: 70; and
(v) the PD-L2 extracellular region has at least 70%, preferably at least 80%, and more preferably at least 90%, 95%, 97% or 99% or 100% sequence identity to an amino acid sequence represented by SEQ ID NO: 71.

12. The NK inhibitory molecule according to claim 1, wherein the transmembrane domain is a transmembrane domain of a protein selected from the group consisting of: TCR α chain, TCR β chain, TCR γ chain, TCR δ chain, CD3 ζ subunit, CD3 ε subunit, CD3 γ subunit, CD3 δ subunit, CD45, CD4, CD5, CD8 α, CD9, CD16, CD22, CD33, CD28, CD37, CD64, CD80, CD86, CD134, CD137, CD154, HLA-E, HLA-F, HLA-G, cadherin, collagen, and OCIL.

13. The NK inhibitory molecule according to claim 1, wherein the co-stimulatory domain is a co-stimulatory signaling domain of a protein selected from the group consisting of LTB, CD94, TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134 (OX40), CD137 (4-1 BB), CD270 (HVEM), CD272 (BTLA), CD276 (B7-H3), CD278 (ICOS), CD357 (GITR), DAP10, DAP12, LAT, NKG2C, SLP76, PD-1, LIGHT, TRIM, ZAP70, and a combination thereof.

14. The NK inhibitory molecule according to claim 1, wherein the NK inhibitory molecule does not contain an intracellular signaling domain.

15. The NK inhibitory molecule according to claim 1, wherein the NK inhibitory molecule further contains an intracellular signaling domain.

16. The NK inhibitory molecule according to claim 14 or 15, wherein the intracellular signaling domain is a signaling domain of a protein selected from the group consisting of: FcR γ, FcR β, CD3 γ, CD3 δ, CD3 ε, CD3 ζ, CD22, CD79a, CD79b, and CD66d.

17. The NK inhibitory molecule according to claim 14 or 15, wherein the intracellular signaling domain comprises a CD3 ζ signaling domain.

18. A nucleic acid molecule, **characterized by** encoding the NK inhibitory molecule according to any one of claims 1-17.

19. A vector, **characterized by** comprising the nucleic acid molecule according to claim 18.

20. An engineered immune cell, **characterized by** (1) expressing the NK inhibitory molecule according to any one of claims 1-17, and (2) with the expression of at least one MHC-related gene being suppressed or silenced.

21. The engineered immune cell according to claim 20, wherein the engineered immune cell further expresses a chimeric antigen receptor, and the chimeric antigen receptor comprises a ligand binding domain, a transmembrane domain, a co-stimulatory domain, and an intracellular signaling domain.

22. An engineered immune cell, **characterized by** (1) expressing a fusion protein of an NK inhibitory molecule and a chimeric antigen receptor, wherein the fusion protein comprises an NK inhibitory ligand, a ligand binding domain, a transmembrane domain, a co-stimulatory domain, and an intracellular signaling domain, and (2) with the expression of at least one MHC-related gene being suppressed or silenced.

23. The engineered immune cell according to any one of claims 20-22, wherein the at least one MHC-related gene is selected from the group consisting of HLA-A, HLA-B, HLA-C, B2M, HLA-DPA, HLA-DQ, HLA-DRA, TAP1, TAP2, LMP2, LMP7, RFX5, RFXAP, RFXANK, CIITA, and a combination thereof.

24. The engineered immune cell according to claim 23, wherein the at least one MHC-related gene is selected from the group consisting of B2M, RFX5, RFXAP, RFXANK, CIITA, and a combination thereof.

25. The engineered immune cell according to claim 24, wherein the MHC-related gene comprises B2M, wherein the NK inhibitory ligand is a fusion molecule of B2M and an extracellular region of non-classical HLA-class I molecule.

26. The engineered immune cell according to claim 25, wherein the non-classical HLA-class I molecule is HLA-E or HLA-G.

27. The engineered immune cell according to claim 25, wherein the NK inhibitory molecule further comprises a presenting peptide, which is selected from SEQ ID NOS: 46-53.

28. The engineered immune cell according to any one of claims 20-27, wherein expression of at least one TCR/CD3 gene of the engineered immune cell is suppressed or silenced, and the TCR/CD3 gene is selected from the group consisting of TRAC, TRBC, CD3 γ, CD3 δ, CD3 ε, CD3 ζ, and a combination thereof.

29. The engineered immune cell according to any one of claims 20-28, wherein expression of one or more genes, selected from the group consisting of: CD52, GR, dCK, PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3, is suppressed or silenced.

30. The engineered immune cell according to any one of claims 20-29, wherein the ligand binding domain binds to a target selected from the group consisting of: TSHR, CD2, CD3, CD4, CD5, CD7, CD8, CD14, CD15, CD19, CD20, CD21, CD23, CD24, CD25, CD37, CD38, CD40, CD40L, CD44, CD46, CD47, CD52, CD54, CD56, CD70, CD73, CD80, CD97, CD123, CD22, CD126, CD138, CD 179a, DR4, DR5, TAC, TEM1/CD248, VEGF, GUCY2C, EGP40, EGP-2, EGP-4, CD133, IFNAR1, DLL3, kappa light chain, TIM3, BAFF-R, CD30, CD171, CS-1, CLL-1, CD33, EGFRvIII, tEGFR, GD2, GD3, BCMA, GPRC5D, Tn antigen, PSMA, ROR1, FLT3, FAP, TAG72, CD38, CD44v6, CEA, EPCAM, B7H3, KIT, IL-13Ra2, IL-22Ra, IL-2, mesothelin, IL-11Ra, PSCA, PRSS21, VEGFR2, LewisY, CD24, PDGFR-β, SSEA-4, CD20, AFP, Folate receptor α, ERBB2 (Her2/neu), ErbB3, ErbB4, MUC1, MUC16, EGFR, CS1, CD138, NCAM, Claudin18.2, Prostase, PAP, ELF2M, Ephrin B2, IGF-I receptor, CAIX, LMP2, gp100, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, MAGE-A3, MAGE-A6, legumain, HPV E6, E7, MAGE-A4, MART-1, WT-1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGFβ, APRIL, NKG2D, NKG2DL, and any combination thereof.

31. The engineered immune cell according to claim 30, wherein the target is selected from the group consisting of CD7, CD19, CD20, CD22, CD30, CD33, CD38, CD123, CD138, CD171, MUC1, AFP, Folate receptor α, CEA, PSCA, PSMA, Her2, EGFR, IL13Ra2, GD2, NKG2D, EGFRvIII, CS1, BCMA, mesothelin, Cluadin18.2, ROR1, NY-ESO-1, MAGE-A4, and any combination thereof.

32. The engineered immune cell according to any one of claims 20-31, wherein the engineered immune cell is a B cell, a T cell, a macrophage, a dendritic cell, a monocyte, an NK cell, or an NKT cell.

33. The engineered immune cell according to claim 32, wherein the engineered immune cell is a CD4+/CD8+ T cell, a CD4+ helper T cell, a CD8+ T cell, a tumor infiltrating cell, a memory T cell, a naive T cell, a γδ-T cell, or an αβ-T cell.

34. A pharmaceutical composition, **characterized by** containing the NK inhibitory molecule according to any one of claims 1-17, the nucleic acid molecule according to claim 18, the vector according to claim 19 or the engineered immune cell according to any one of claims 20-33, and one or more pharmaceutically acceptable excipients.

35. Use of the NK inhibitory molecule according to any one of claims 1-17, the nucleic acid molecule according to claim 18, the vector according to claim 19 or the engineered immune cell according to any one of claims 20-33 in preparation of a medicine for treating cancers, infections or autoimmune diseases.
